# EUROPEAN PATENT APPLICATION

(11) **EP 4 782 444 A1**
(43) Date of publication of application: **29.07.2026**
(21) Application number: 23952468.9
(22) Date of filing: 18.09.2023
(51) Int. Cl.: C07D 403/14, C07D 487/00, A61P 35/00

(54) **CRYSTAL FORM OF PYRIMIDINE ANILINE COMPOUND, PREPARATION METHOD, AND USE**

(71) Applicant: TYK Medicines, Inc., Huzhou, Zhejiang 313100 (CN); TYK Medicines (Zhengzhou), Inc., Zhengzhou, Henan 451162 (CN)
(72) Inventor: LI, Jun, Huzhou, Zhejiang 313100 (CN); NIU, Chengshan, Zhengzhou, Henan 451162 (CN); GUO, Zhongwei, Zhengzhou, Henan 451162 (CN); LIANG, Apeng, Huzhou, Zhejiang 313100 (CN); WU, Yusheng, Huzhou, Zhejiang 313100 (CN)
(74) Representative: dompatent
(86) International application number: PCT/CN2023/119440
(87) International publication number: WO 2025/059801

(57) **Abstract**

The present invention relates to a polymorphic form of an acid salt of a pyrimidine aniline compound, a preparation method, and a use. Specifically, disclosed in the present invention are a polymorphic form of an acid salt of a compound of formula (A), a preparation method for the polymorphic form, and a use of the polymorphic form. Taking into account a thermodynamic stability relationship and a solid-state property evaluation result, the present invention provides a citrate crystal form I of formula (A) as a preferred crystal form, and the citrate crystal form I has stable physicochemical properties and can be better used clinically.

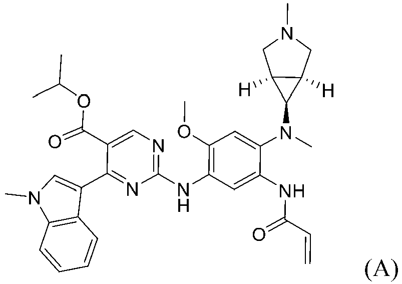

## Description

### FIELD OF THE INVENTION

The present invention belongs to the field of medicinal chemistry, and specifically relates to the crystal forms of a compound used as kinase inhibitor, the preparation method therefor, and use thereof.

### BACKGROUND OF THE INVENTION

Epidermal growth factor receptor (EGFR) belongs to the family of receptor tyrosine kinase (RTK), which includes EGFR/ERBB1, HER2/ERBB2/NEU, HER3/ERBB3, and HER4/ERBB4. Epidermal growth factor receptor activates its tyrosine kinase activity through homodimerization or heterodimerization, then phosphorylates its substrate, thereby activating several related downstream pathways in cells, such as the PI3K-AKT-mTOR pathway involved in cell survival and the RAS-RAF-MEK-ERK pathway involved in cell proliferation. Mutations or amplification of the epidermal growth factor receptor both can lead to activation of the epidermal growth factor receptor kinase, resulting in the occurrence of a variety of human diseases, such as malignant tumors. For example, in non-small cell lung cancer patients, more than 10% of the American patients have EGFR mutations, while the percentage of Asian patients with EGFR mutations can reach nearly 50%. Meanwhile, the incidence rate of HER2 mutations in non-small cell lung cancer patients is about 2-4%.

EGFR mutations mainly include deletions, insertions and point mutations, wherein the deletion of exon 19 and L858R point mutation of exon 21 account for nearly 90% of EGFR mutations. For tumor patients with these EGFR mutations, the currently commercially available EGFR-TKIs include the first-generation Iressa, Tarceva, and Conmana, the second-generation Afatinib and Dacomitinib, and the third-generation Osimertinib. The other 10% of EGFR mutations primarily involve exons 18 and 20 of the EGFR, and the exon 20 insertion mutation of EGFR accounts for about 9% of the overall EGFR mutations. The most common HER2 mutation in tumor patients with HER2 mutation is the exon 20 insertion mutation of HER2.

TAK-788 is therapeutically effective for exon 20 insertion mutations of EGFR and HER2, and the compound has been marketed in the U.S.. According to its reported clinical trial results, the objective response rate is 43%. DZD9008 has recently been reported to be effective for use in treating advanced non-small cell lung cancer with EGFR or HER2 mutations, with an objective response rate of 40% for EGFR exon 20ins, but the efficacy is not satisfactory.

Therefore, developing novel compounds with EGFR/HER2 kinase inhibitory activity and improved pharmacodynamic and pharmacokinetic properties has become an important research project in the development of new anti-tumor drugs, which are ultimately used for the treatment of diseases such as human tumor.

The application with application number PCT/CN2023/081557 discloses a series of compounds used as EGFR/HER2 kinase inhibitors, and the series of compounds disclosed in this application exhibit excellent EGFR/HER2 inhibitory activity. In the application, a compound represented by Formula (A) is disclosed.

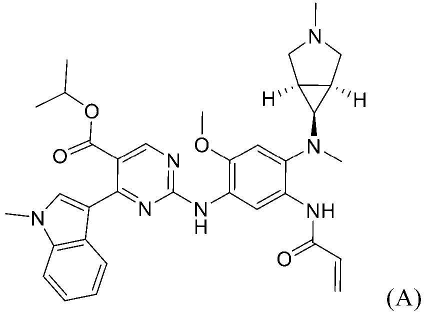

To date, no patents regarding the crystalline forms of the acid salt of this compound have been reported. It is of great significance for subsequent progress for screening and development of crystalline forms of the compound represented by Formula (A) and identifying stable and reliable crystalline forms, so as to ensure the stability of quality of the compound represented by Formula (A), to facilitate its better application in pharmacy, pharmaceutical preparations and future clinical use, and to achieve stable and controllable drug quality, better dissolution and higher bioavailability, thereby avoiding safety issues such as side effects caused by toxic impurities as the result of drug instability.

### SUMMARY OF THE INVENTION

The purpose of the present invention is to provide acid salt crystalline forms of pyrimidine aniline compounds, as well as the preparation method therefor and the use thereof.

In the first aspect of the present invention, provided is an acid salt crystalline form of a compound of Formula A, the salt crystalline form is selected from the group consisting of: citrate crystalline form I, maleate crystalline form I, maleate crystalline form II, oxalate crystalline form I, oxalate crystalline form II, mesylate crystalline form I, and mesylate crystalline form II, 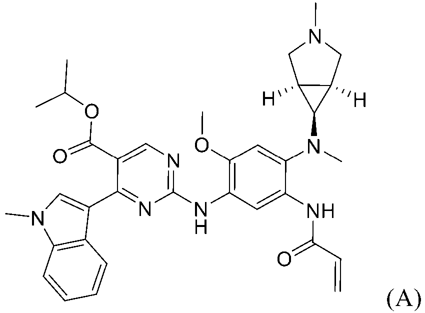 wherein,
the X-ray powder diffraction pattern of the citrate crystalline form I comprises characteristic peaks at 3 or more (e.g., 4, or 5) 20 values selected from the group consisting of: 6.9±0.2°, 7.9±0.2°, 13.3±0.2°, 16.0±0.2°, and 18.6±0.2°.

In another preferred embodiment, the X-ray powder diffraction pattern of the citrate crystalline form I comprises characteristic peaks at 5 or more (e.g., 6, 7, 8, or 9) 2θ values selected from the group consisting of: 6.9±0.2°, 7.9±0.2°, 11.4±0.2°, 13.3±0.2°, 15.7±0.2°, 16.0±0.2°, 16.7±0.2°, 18.6±0.2°, 19.2±0.2°, and 20.8±0.2°.

In another preferred embodiment, the X-ray powder diffraction pattern of the citrate crystalline form I comprises characteristic peaks at 6 or more (e.g., 7, 8, 9, or 10) 2θ values selected from the group consisting of: 6.9±0.2°, 7.9±0.2°, 11.4±0.2°, 13.3±0.2°, 14.9±0.2°, 15.7±0.2°, 16.0±0.2°, 16.7±0.2°, 17.2±0.2°, 18.6±0.2°, 19.2±0.2°, 20.8±0.2°, 21.0±0.2°, 22.9±0.2°, 23.8±0.2°, and 27.1±0.2°.

In another preferred embodiment, the X-ray powder diffraction pattern of the citrate crystalline form I has characteristic peaks at the following 20 values: 3.5±0.2°, 6.9±0.2°, 7.4±0.2°, 7.9±0.2°, 10.0±0.2°, 10.3±0.2°, 10.7±0.2°, 11.4±0.2°, 13.3±0.2°, 13.9±0.2°, 14.9±0.2°, 15.7±0.2°, 16.0±0.2°, 16.7±0.2°, 17.2±0.2°, 17.8±0.2°, 18.6±0.2°, 19.2±0.2°, 20.8±0.2°, 21.0±0.2°, 21.4±0.2°, 21.7±0.2°, 22.9±0.2°, 23.8±0.2°, 24.8±0.2°, 25.8±0.2°, 27.1±0.2°, 27.3±0.2°, 27.6±0.2°, and 28.5±0.2°.

In another preferred embodiment, the citrate crystalline form I has X-ray powder diffraction data substantially as shown in Table 5.

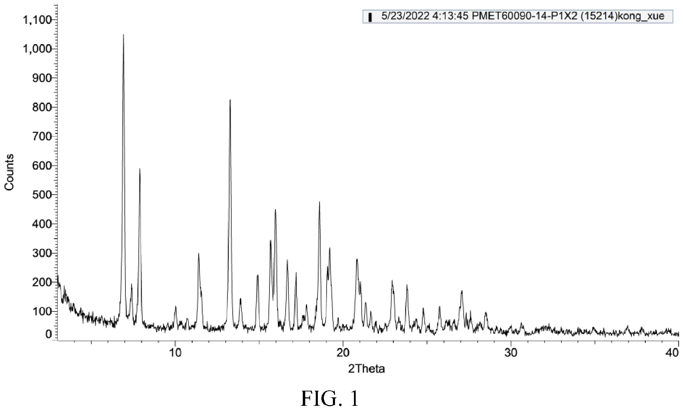

In another preferred embodiment, the X-ray powder diffraction pattern of the citrate crystalline form I is substantially as shown in Figure 1.

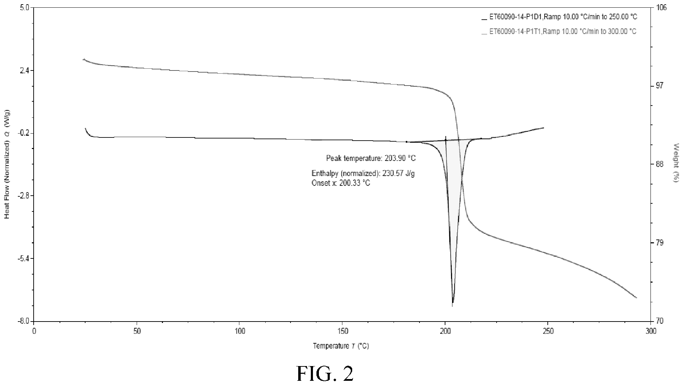

In another preferred embodiment, the thermogravimetric analysis pattern of the citrate crystalline form I is substantially as shown in Figure 2.

In another preferred embodiment, the differential scanning calorimetry pattern of the citrate crystalline form I is substantially as shown in Figure 2.

In another preferred embodiment, the citrate crystalline form I exhibits an endothermic peak at an onset temperature of 200.33±2°C in differential scanning calorimetry test.

In another preferred embodiment, the citrate crystalline form I shows significant weight loss at 150°C-230°C (preferably 160°C-220°C), with a weight loss of more than 5%, preferably more than 10%, more preferably more than 15%.

In another preferred embodiment, the weight loss is the result of decomposition, and there is no significant weight loss before decomposition.

In another preferred embodiment, the residual acetone solvent in the citrate crystalline form I is less than 2%, preferably less than 1%.

In another preferred embodiment, the citrate crystalline form I is an anhydrous crystalline form.

In another preferred embodiment, in the citrate crystalline form I, the molar ratio of the compound of Formula A to citric acid is (0.8-1.2):(0.8-1.2), preferably (0.9-1.1):(0.9-1.1), more preferably (0.95-1.05):(0.95-1.05), e.g., 1:1.05, 1:1.

In another preferred embodiment, the citrate crystalline form I is triclinic system, space group P1.

In another preferred embodiment, the unit cell parameters of the citrate crystalline form I are a = 11.9331(2) Å, b = 13.4684(2) Å, c = 13.6474(2) Å, α = 69.2800(10)°, β = 82.1490(10)°, γ = 85.5730(10)°, V = 2031.32(6) Å³, Z = 2.

The X-ray powder diffraction pattern of the maleate crystalline form I comprises characteristic peaks at 3 or more (e.g., 4, or 5) 2θ values selected from the group consisting of: 6.7±0.2°, 9.6±0.2°, 14.3±0.2°, 14.6±0.2°, and 23.5±0.2°.

In another preferred embodiment, the X-ray powder diffraction pattern of the maleate crystalline form I comprises characteristic peaks at 5 or more (e.g., 6, 7, 8, 9, or 10) 2θ values selected from the group consisting of: 6.7±0.2°, 8.1±0.2°, 9.6±0.2°, 14.3±0.2°, 14.6±0.2°, 15.8±0.2°, 16.4±0.2°, 20.2±0.2°, 22.4±0.2°, and 23.5±0.2°.

In another preferred embodiment, the X-ray powder diffraction pattern of the maleate crystalline form I comprises characteristic peaks at 6 or more (e.g., 7, 8, 9, 10, 11 or 12) 2θ values selected from the group consisting of: 6.7±0.2°, 8.1±0.2°, 9.6±0.2°, 14.3±0.2°, 14.6±0.2°, 15.8±0.2°, 16.4±0.2°, 19.4±0.2°, 19.9±0.2°, 20.2±0.2°, 21.4±0.2°, 22.4±0.2°, 23.5±0.2°, and 24.6±0.2°.

In another preferred embodiment, the X-ray powder diffraction pattern of the maleate crystalline form I has characteristic peaks at the following 2θ values: 6.7±0.2°, 7.8±0.2°, 8.1±0.2°, 9.6±0.2°, 14.3±0.2°, 14.6±0.2°, 15.8±0.2°, 16.4±0.2°, 18.7±0.2°, 19.0±0.2°, 19.4±0.2°, 19.9±0.2°, 20.2±0.2°, 21.4±0.2°, 22.4±0.2°, 23.1±0.2°, 23.5±0.2°, 24.6±0.2°, 25.5±0.2°, and 26.1±0.2°.

In another preferred embodiment, the maleate crystalline form I has X-ray powder diffraction data substantially as shown in Table 6.

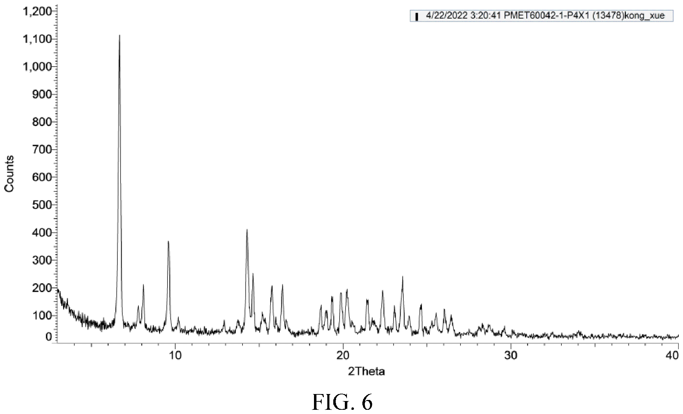

In another preferred embodiment, the X-ray powder diffraction pattern of the maleate crystalline form I is substantially as shown in Figure 6.

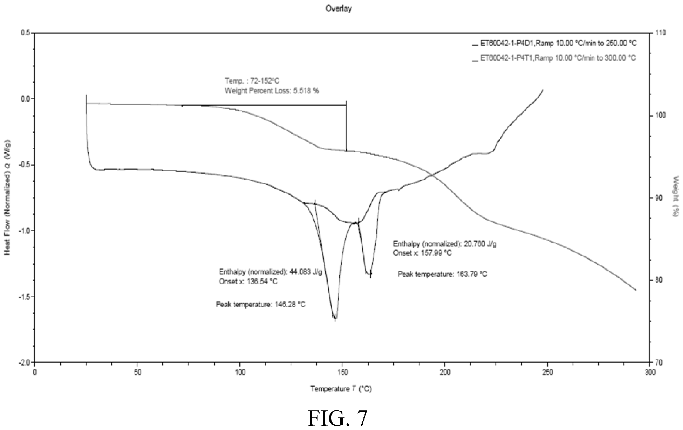

In another preferred embodiment, the thermogravimetric analysis pattern of the maleate crystalline form I is substantially as shown in Figure 7.

In another preferred embodiment, the differential scanning calorimetry pattern of the maleate crystalline form I is substantially as shown in Figure 7.

In another preferred embodiment, the maleate crystalline form I exhibits endothermic peaks at onset temperature of 136.54±2°C and 157.99±2°C respectively in differential scanning calorimetry test.

In another preferred embodiment, the maleate crystalline form I shows significant weight loss at 60°C-170°C (preferably 70°C-160°C), with a weight loss of more than 3%, preferably more than 5%.

In another preferred embodiment, the residual acetone solvent in the maleate crystalline form I is more than 3%, preferably more than 5%.

In another preferred embodiment, the maleate crystalline form I is a solvate.

In another preferred embodiment, in the maleate crystalline form I, the molar ratio of the compound of Formula A to maleic acid is (0.8-1.2):(0.8-1.2), preferably (0.9-1.1):(0.9-1.1), more preferably (0.95-1.05):(0.95-1.05), e.g., 1:1.05, 1:1.

The X-ray powder diffraction pattern of the maleate crystalline form II comprises characteristic peaks at 3 or more (e.g., 4, or 5) 20 values selected from the group consisting of: 6.9±0.2°, 10.6±0.2°, 14.5±0.2°, 16.5±0.2°, and 17.0±0.2°.

In another preferred embodiment, the X-ray powder diffraction pattern of the maleate crystalline form II comprises characteristic peaks at 5 or more (e.g., 6, 7, 8, 9 or 10) 20 values selected from the group consisting of: 6.9±0.2°, 10.6±0.2°, 14.5±0.2°, 15.5±0.2°, 16.14+0.2°, 16.5±0.2°, 17.0±0.2°, 19.4±0.2°, 21.5±0.2°, and 22.0±0.2°.

In another preferred embodiment, the X-ray powder diffraction pattern of the maleate crystalline form II comprises characteristic peaks at 6 or more (e.g., 7, 8, 9, 10, 11 or 12) 2θ values selected from the group consisting of: 6.9±0.2°, 9.8±0.2°, 10.6±0.2°, 14.5±0.2°, 15.5±0.2°, 16.1±0.2°, 16.5±0.2°, 17.0±0.2°, 19.4±0.2°, 21.5±0.2°, 22.0±0.2°, 23.1±0.2°, 25.2±0.2°, 25.8±0.2°, and 27.3±0.2°.

In another preferred embodiment, the X-ray powder diffraction pattern of the maleate crystalline form II has characteristic peaks at the following 2θ values: 6.9±0.2°, 9.8±0.2°, 10.6±0.2°, 14.5±0.2°, 15.5±0.2°, 16.1±0.2°, 16.5±0.2°, 17.0±0.2°, 19.4±0.2°, 21.1±0.2°, 21.5±0.2°, 22.0±0.2°, 23.1±0.2°, 24.2±0.2°, 25.2±0.2°, 25.8±0.2°, and 27.3±0.2°.

In another preferred embodiment, the maleate crystalline form II has X-ray powder diffraction data substantially as shown in Table 7.

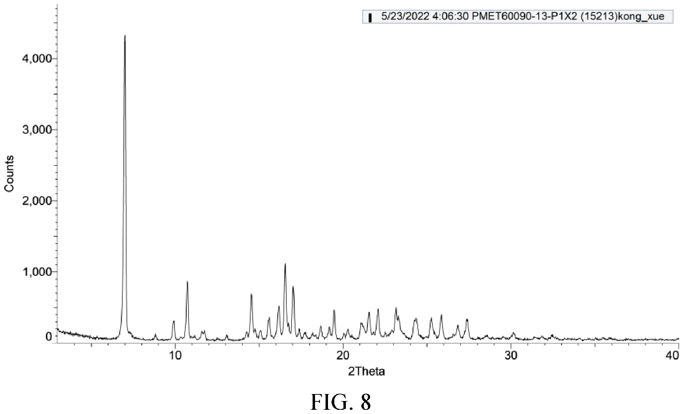

In another preferred embodiment, the X-ray powder diffraction pattern of the maleate crystalline form II is substantially as shown in Figure 8.

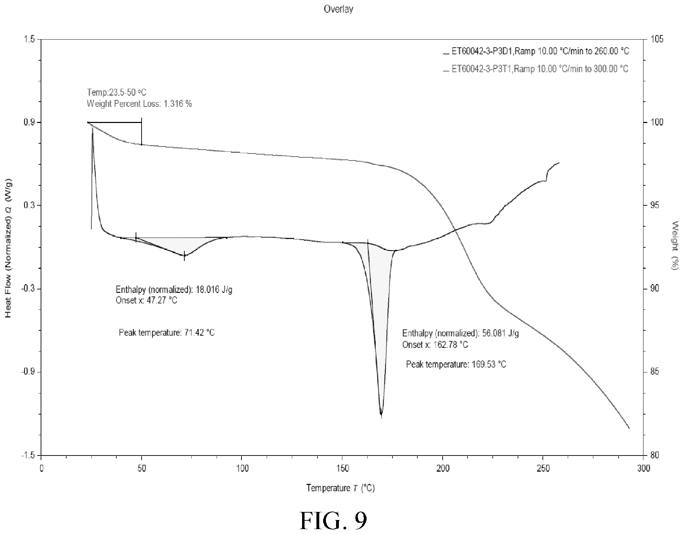

In another preferred embodiment, the thermogravimetric analysis pattern of the maleate crystalline form II is substantially as shown in Figure 9.

In another preferred embodiment, the differential scanning calorimetry pattern of the maleate crystalline form II is substantially as shown in Figure 9.

In another preferred embodiment, the maleate crystalline form II exhibits endothermic peaks at onset temperature of 47.27±2°C and 162.78±2°C respectively in differential scanning calorimetry test.

In another preferred embodiment, the maleate crystalline form II shows weight loss at 15°C-60°C (preferably 20°C-55°C), with a weight loss of more than 0.5%, preferably more than 1%.

In another preferred example, the maleate crystalline form II shows weight loss at 15°C-60°C (preferably 20°C-55°C), corresponding to a corresponding endothermic peak at 45-75 °C in differential scanning calorimetry test.

In another preferred embodiment, the residual methyl *tert-butyl* ether solvent in the maleate crystalline form II is less than 2%, preferably less than 1%.

In another preferred embodiment, the maleate crystalline form II remains stable at 90 - 130 °C without undergoing any crystal form transformation.

In another preferred embodiment, the maleate crystalline form II is a hydrate or an easily hygroscopic anhydrous crystalline form.

In another preferred embodiment, in the maleate crystalline form II, the molar ratio of the compound of Formula A to maleic acid is (0.8-1.2):(0.8-1.2), preferably (0.9-1.1):(0.9-1.1), more preferably (0.95-1.05):(0.95-1.05), e.g., 1:1.05, 1:1.

The X-ray powder diffraction pattern of the oxalate crystalline form I comprises characteristic peaks at 3 or more (e.g., 4, or 5) 20 values selected from the group consisting of: 9.4±0.2°, 12.0±0.2°, 12.7±0.2°, 13.7±0.2°, and 21.4±0.2°.

In another preferred embodiment, the X-ray powder diffraction pattern of the oxalate crystalline form I comprises characteristic peaks at 5 or more (e.g., 6, 7, 8, 9, 10 or 11) 20 values selected from the group consisting of: 6.8±0.2°, 9.4±0.2°, 12.0±0.2°, 12.7±0.2°, 13.7±0.2°, 14.0±0.2°, 19.9±0.2°, 20.6±0.2°, 21.4±0.2°, 22.5±0.2°, and 24.9±0.2°.

In another preferred embodiment, the X-ray powder diffraction pattern of the oxalate crystalline form I comprises characteristic peaks at 6 or more (e.g., 7, 8, 9, or 10) 2θ values selected from the group consisting of: 6.8±0.2°, 9.0±0.2°, 9.4±0.2°, 10.6±0.2°, 12.0±0.2°, 12.7±0.2°, 13.7±0.2°, 14.0±0.2°, 14.2±0.2°, 15.3±0.2°, 19.1±0.2°, 19.9±0.2°, 20.6±0.2°, 21.4±0.2°, 22.5±0.2°, 23.6±0.2°, and 24.9±0.2°.

In another preferred embodiment, the X-ray powder diffraction pattern of the oxalate crystalline form I has characteristic peaks at the following 2θ values: 6.8±0.2°, 9.0±0.2°, 9.4±0.2°, 10.6±0.2°, 12.0±0.2°, 12.7±0.2°, 13.7±0.2°, 14.0±0.2°, 14.2±0.2°, 15.3±0.2°, 19.1±0.2°, 19.9±0.2°, 20.6±0.2°, 21.4±0.2°, 22.5±0.2°, 22.9±0.2°, 23.2±0.2°, 23.6±0.2°, and 24.9±0.2°.

In another preferred embodiment, the oxalate crystalline form I has X-ray powder diffraction data substantially as shown in Table 8.

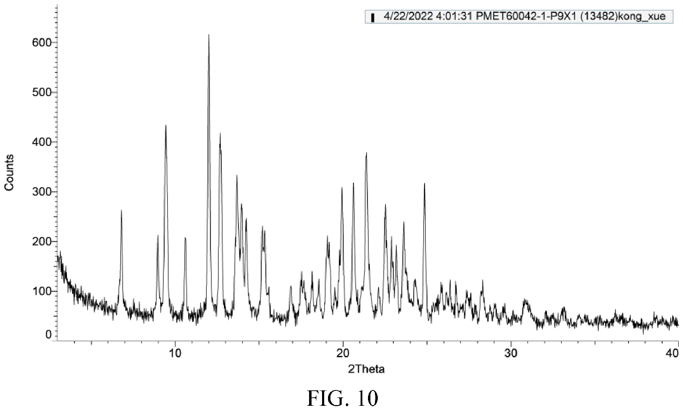

In another preferred embodiment, the X-ray powder diffraction pattern of the oxalate crystalline form I is substantially as shown in Figure 10.

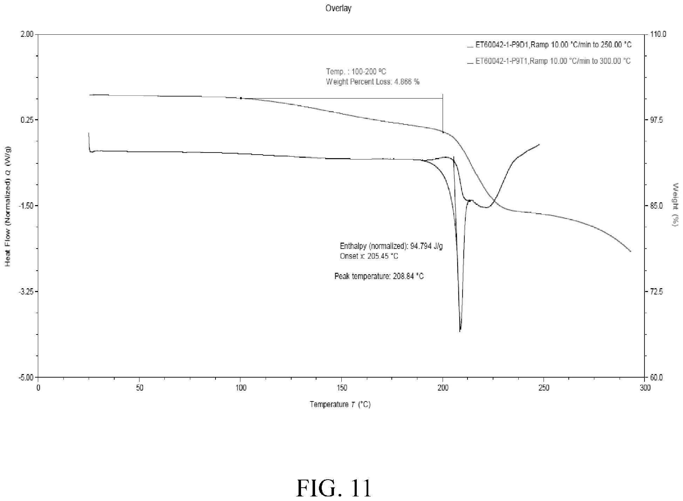

In another preferred embodiment, the thermogravimetric analysis pattern of the oxalate crystalline form I is substantially as shown in Figure 11.

In another preferred embodiment, the differential scanning calorimetry pattern of the oxalate crystalline form I is substantially as shown in Figure 11.

In another preferred embodiment, the oxalate crystalline form I exhibits an endothermic peak at onset temperature of 205.45±2°C in differential scanning calorimetry test.

In another preferred embodiment, the oxalate crystalline form I shows weight loss at 80°C-220°C (preferably 90°C-210°C), with a weight loss of more than 3%, preferably more than 4%.

In another preferred embodiment, the residual acetone solvent in the oxalate crystalline form I is more than 20%, preferably more than 30%.

In another preferred embodiment, the oxalate crystalline form I is an acetone solvate.

In another preferred example, the oxalate crystalline form I transforms into oxalate crystalline form II after heating to 180-200 °C.

In another preferred embodiment, in the oxalate crystalline form I, the molar ratio of the compound of Formula A to oxalic acid is (0.8-1.2):(0.8-1.2), preferably (0.9-1.1):(0.9-1.1), more preferably (0.95-1.05):(0.95-1.05), e.g., 1:1.05, 1:1.

The X-ray powder diffraction pattern of the oxalate crystalline form II comprises characteristic peaks at 3 or more (e.g., 4, 5, 6, or 7) 20 values selected from the group consisting of: 9.5±0.2°, 12.2±0.2°, 12.7±0.2°, 13.7±0.2°, 14.5±0.2°, 19.9±0.2°, and 24.8±0.2°.

In another preferred embodiment, the X-ray powder diffraction pattern of the oxalate crystalline form II comprises characteristic peaks at 5 or more (e.g., 6, 7, 8, 9, 10 or 11) 2θ values selected from the group consisting of: 9.1±0.2°, 9.5±0.2°, 10.6±0.2°, 12.2±0.2°, 12.7±0.2°, 13.7±0.2°, 14.3±0.2°, 14.5±0.2°, 19.9±0.2°, 21.9±0.2°, 23.1±0.2°, and 24.8±0.2°.

In another preferred embodiment, the X-ray powder diffraction pattern of the oxalate crystalline form II comprises characteristic peaks at 6 or more (e.g., 7, 8, 9, 10, 11 or 12) 2θ values selected from the group consisting of: 6.8±0.2°, 9.1±0.2°, 9.5±0.2°, 10.6±0.2°, 12.2±0.2°, 12.7±0.2°, 13.7±0.2°, 13.8±0.2°, 14.3±0.2°, 14.5±0.2°, 19.9±0.2°, 21.1±0.2°, 21.9±0.2°, 23.1±0.2°, 23.8±0.2°, and 24.8±0.2°.

In another preferred embodiment, the X-ray powder diffraction pattern of the oxalate crystalline form II has characteristic peaks at the following 2θ values: 6.8±0.2°, 9.1±0.2°, 9.5±0.2°, 10.6±0.2°, 12.2±0.2°, 12.7±0.2°, 13.7±0.2°, 13.8±0.2°, 14.3±0.2°, 14.5±0.2°, 15.2±0.2°, 17.2±0.2°, 19.1±0.2°, 19.3±0.2°, 19.9±0.2°, 21.1±0.2°, 21.9±0.2°, 23.1±0.2°, 23.8±0.2°, and 24.8±0.2°.

In another preferred embodiment, the oxalate crystalline form II has X-ray powder diffraction data substantially as shown in Table 9.

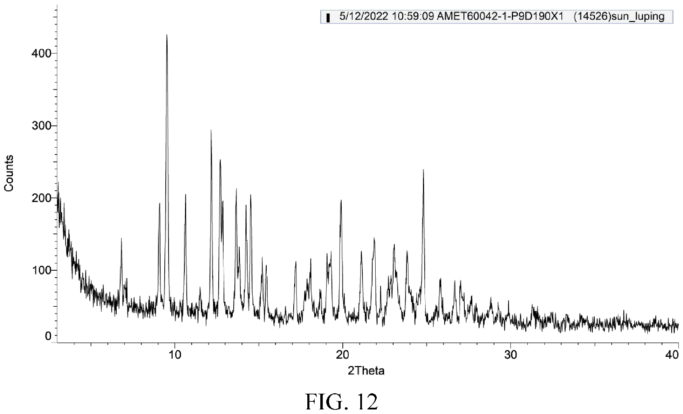

In another preferred embodiment, the X-ray powder diffraction pattern of the oxalate crystalline form II is substantially as shown in Figure 12.

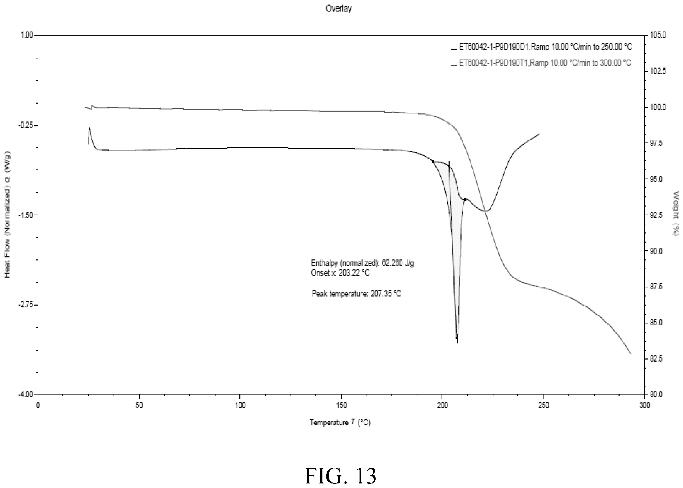

In another preferred embodiment, the thermogravimetric analysis pattern of the oxalate crystalline form II is substantially as shown in Figure 13.

In another preferred embodiment, the differential scanning calorimetry pattern of the oxalate crystalline form II is substantially as shown in Figure 13.

In another preferred embodiment, the oxalate crystalline form II exhibits an endothermic peak at onset temperature of 203.22±2°C in differential scanning calorimetry test.

In another preferred embodiment, the oxalate crystalline form II shows no significant weight loss before decomposition.

In another preferred embodiment, the oxalate crystalline form II is an anhydrous crystalline form.

In another preferred embodiment, in the oxalate crystalline form II, the molar ratio of the compound of Formula A to oxalic acid is (0.8-1.2):(0.8-1.2), preferably (0.9-1.1):(0.9-1.1), more preferably (0.95-1.05):(0.95-1.05), e.g., 1:1.05, 1:1.

The X-ray powder diffraction pattern of the mesylate crystalline form I comprises characteristic peaks at 3 or more (e.g., 4, 5, or 6) 20 values selected from the group consisting of: 11.0±0.2°, 16.7±0.2°, 19.2±0.2°, 22.1±0.2°, 23.4±0.2°, and 25.0±0.2°.

In another preferred embodiment, the X-ray powder diffraction pattern of the mesylate crystalline form I comprises characteristic peaks at 5 or more (e.g., 6, 7, 8, 9, 10 or 11) 20 values selected from the group consisting of: 8.8±0.2°, 10.2±0.2°, 11.0±0.2°, 15.6±0.2°, 16.7±0.2°, 17.0±0.2°, 18.5±0.2°, 19.2±0.2°, 22.1±0.2°, 23.4±0.2°, and 25.0±0.2°.

In another preferred embodiment, the X-ray powder diffraction pattern of the mesylate crystalline form I comprises characteristic peaks at 6 or more (e.g., 7, 8, 9, 10, 11 or 12) 2θ values selected from the group consisting of: 8.8±0.2°, 10.2±0.2°, 11.0±0.2°, 15.6±0.2°, 16.7±0.2°, 17.0±0.2°, 17.6±0.2°, 18.5±0.2°, 19.2±0.2°, 19.6±0.2°, 20.3±0.2°, 21.5±0.2°, 22.1±0.2°, 22.7±0.2°, 23.4±0.2°, and 25.0±0.2°.

In another preferred embodiment, the X-ray powder diffraction pattern of the mesylate crystalline form I has characteristic peaks at the following 2θ values: 8.8±0.2°, 10.2±0.2°, 11.0±0.2°, 15.6±0.2°, 16.7±0.2°, 17.0±0.2°, 17.6±0.2°, 18.5±0.2°, 19.2±0.2°, 19.6±0.2°, 20.3±0.2°, 21.5±0.2°, 22.1±0.2°, 22.7±0.2°, 23.4±0.2°, 25.0±0.2°, and 29.7±0.2°.

In another preferred embodiment, the mesylate crystalline form I has X-ray powder diffraction data substantially as shown in Table 10.

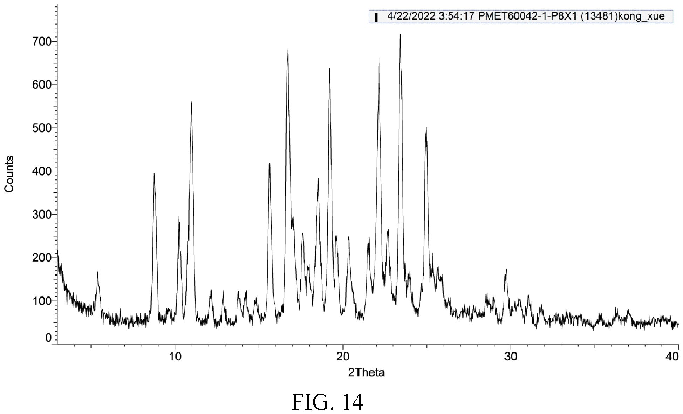

In another preferred embodiment, the X-ray powder diffraction pattern of the mesylate crystalline form I is substantially as shown in Figure 14.

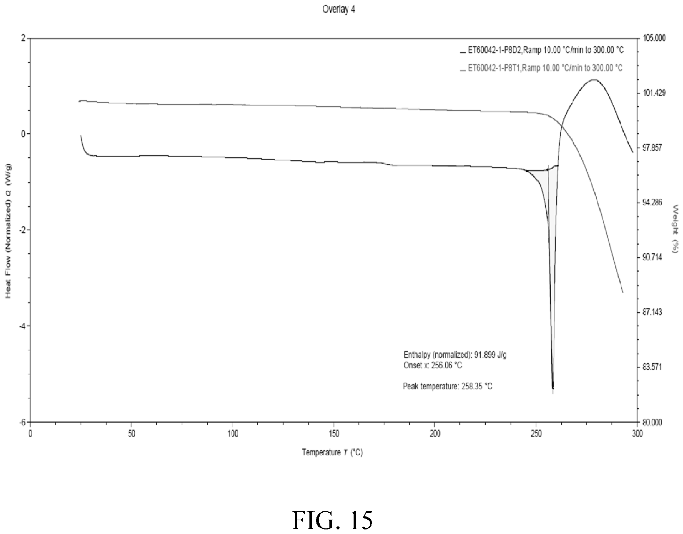

In another preferred embodiment, the thermogravimetric analysis pattern of the mesylate crystalline form I is substantially as shown in Figure 15.

In another preferred embodiment, the differential scanning calorimetry pattern of the mesylate crystalline form I is substantially as shown in Figure 15.

In another preferred embodiment, the mesylate crystalline form I exhibits an endothermic peak at onset temperature of 256.06±2°C in differential scanning calorimetry test.

In another preferred embodiment, the mesylate crystalline form I shows no significant weight loss before decomposition.

In another preferred embodiment, the residual acetone solvent in the mesylate crystalline form I is no more than 2%, preferably no more than 1%.

In another preferred embodiment, the mesylate crystalline form I is an anhydrouscrystalline form.

In another preferred embodiment, in the mesylate crystalline form I, the molar ratio of the compound of Formula A to methanesulfonic acid is (0.9-1.1):(0.9-1.1), preferably (0.95-1.05):(0.95-1.05), e.g., 1:1.05, 1:1.

The X-ray powder diffraction pattern of the mesylate crystalline form II comprises characteristic peaks at 3 or more (e.g., 4, 5, or 6) 2θ values selected from the group consisting of: 7.3±0.2°, 8.8±0.2°, 9.9±0.2°, 10.9±0.2°, 13.1±0.2°, and 14.2±0.2°.

In another preferred embodiment, the X-ray powder diffraction pattern of the mesylate crystalline form II comprises characteristic peaks at 5 or more (e.g., 6, 7, 8, 9, or 10) 20 values selected from the group consisting of: 7.2±0.2°, 7.3±0.2°, 8.8±0.2°, 9.9±0.2°, 10.9±0.2°, 13.1±0.2°, 14.2±0.2°, 16.6±0.2°, 16.9±0.2°, and 22.1±0.2°.

In another preferred embodiment, the X-ray powder diffraction pattern of the mesylate crystalline form II comprises characteristic peaks at 6 or more (e.g., 7, 8, 9, 10, 11 or 12) 2θ values selected from the group consisting of: 7.2±0.2°, 7.3±0.2°, 8.8±0.2°, 9.9±0.2°, 10.9±0.2°, 12.5±0.2°, 13.1±0.2°, 14.2±0.2°, 14.9±0.2°, 16.6±0.2°, 16.9±0.2°, 22.1±0.2°, 22.6±0.2°, and 23.4±0.2°.

In another preferred embodiment, the X-ray powder diffraction pattern of the mesylate crystalline form II has characteristic peaks at the following 20 values: 7.2±0.2°, 7.3±0.2°, 8.8±0.2°, 9.4±0.2°, 9.9±0.2°, 10.9±0.2°, 12.5±0.2°, 13.1±0.2°, 14.2±0.2°, 14.9±0.2°, 16.6±0.2°, 16.9±0.2°, 17.3±0.2°, 19.3±0.2°, 21.4±0.2°, 22.1±0.2°, 22.6+0.2°, and 23.4±0.2°.

In another preferred embodiment, the mesylate crystalline form II has X-ray powder diffraction data substantially as shown in Table 11.

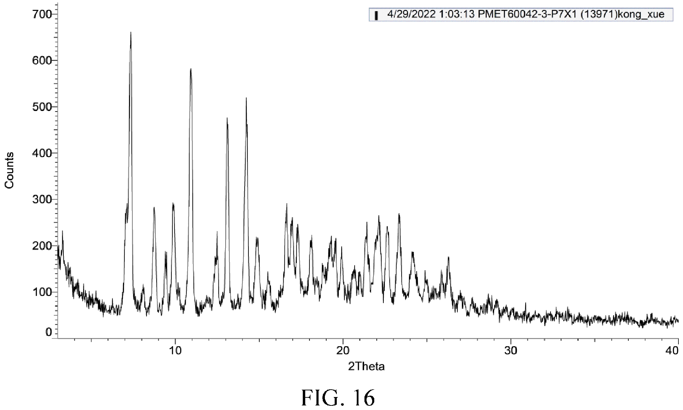

In another preferred embodiment, the X-ray powder diffraction pattern of the mesylate crystalline form II is substantially as shown in Figure 16.

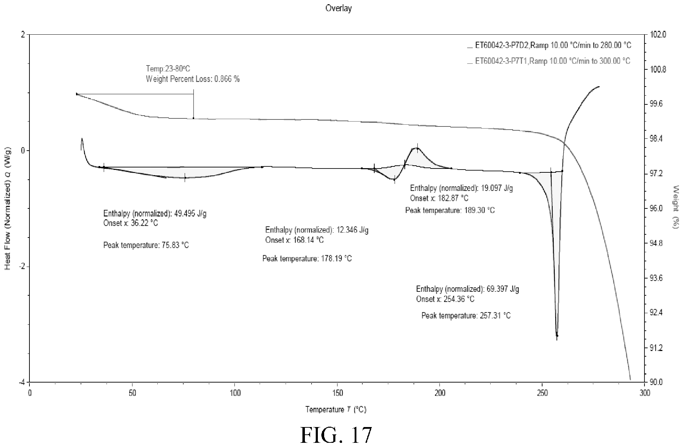

In another preferred embodiment, the thermogravimetric analysis pattern of the mesylate crystalline form II is substantially as shown in Figure 17.

In another preferred embodiment, the differential scanning calorimetry pattern of the mesylate crystalline form II is substantially as shown in Figure 17.

In another preferred embodiment, in the differential scanning calorimetry test of the mesylate crystalline form II, there is an endothermic peak at 35-120 °C (peak value of about 75.83 ± 2 °C), which is the dehydration peak, a set of crystalline transition peaks at 160-200 °C, and an endothermic peak at 240-270 °C, which is the melting peak of the mesylate crystalline form I after crystalline transition.

In another preferred embodiment, the residual methyl *tert-butyl* ether solvent in the mesylate crystalline form II is no more than 2%, preferably no more than 1%, more preferably no more than 0.5%.

In another preferred embodiment, the mesylate crystalline form II is an anhydrous crystalline form.

In another preferred embodiment, in the mesylate crystalline form II, the molar ratio of the compound of Formula A to methanesulfonic acid is (0.9-1.1):(0.9-1.1), preferably (0.95-1.05):(0.95-1.05), e.g., 1:1.05, 1:1.

In another preferred embodiment, the crystalline form is citrate crystalline form I.

In the second aspect of the present invention, provided is a method for preparing the acid salt crystalline form of the compound of Formula A as described in the first aspect of the present invention, wherein the method is selected from the group consisting of:
method 1:
   mixing the compound of Formula A with a first solvent, adding a first acid reagent, and crystallizing out; the first solvent is one selected from the group consisting of methanol, acetone, and acetonitrile;
method 2:
   (a) mixing the compound of Formula A with a second solvent, then mixing with a second acid reagent to obtain an acid salt of the compound of Formula A; the second solvent is one or more selected from the group consisting of 1,2-dichloroethane, methanol, acetone, and acetonitrile;
   (b) mixing the acid salt of the compound of Formula A with a third solvent, subjecting to polymorphic transition in suspension, and crystallizing out; the third solvent is one or more selected from the group consisting of dichloromethane, acetone, tetrahydrofuran, acetonitrile, and methyl *tert-butyl* ether;
and method 3:
   (c) heating an acid salt crystalline form of the compound of Formula A to a certain temperature, performing polymorphic transition to obtain another crystalline form.

In another preferred embodiment, the compound of Formula A is a free base crystalline form I.

In another preferred embodiment, the ratio of the compound of Formula A to the second solvent is 50-250 g/L.

In another preferred embodiment, the first acid reagent and the second acid reagent are each independently selected from the group consisting of: citric acid, oxalic acid, maleic acid, methanesulfonic acid, fumaric acid, L-tartaric acid, hydrochloric acid, L-malic acid, acetic acid, benzoic acid, phosphoric acid, and succinic acid.

In another preferred embodiment, the second solvent is one or more selected from 1,2-dichloroethane and methanol.

In another preferred embodiment, the third solvent is one selected from acetone and methyl *tert-butyl* ether.

In another preferred embodiment, the method comprises:
(a1) mixing the compound of Formula A with a second solvent, then mixing with citric acid to obtain citrate of the compound of Formula A; the second solvent is one or more selected from the group consisting of 1,2-dichloroethane and methanol;
(b1) mixing the citrate of the compound of Formula A with a third solvent, subjecting to polymorphic transition in suspension, and crystallizing out to obtain citrate crystalline form I of the compound of Formula A; the third solvent is acetone.

In another preferred embodiment, the molar ratio of the compound of Formula A to citric acid is (0.8-1.2):(0.8-1.2), preferably (0.9-1.1):(0.9-1.1), more preferably (0.95-1.05):(0.95-1.05), e.g., 1:1.05, 1:1.

In another preferred embodiment, the step (a1) is performed at 0-10°C, e.g., 5°C.

In another preferred embodiment, the salt-forming reaction time of the step (a1) is 8-20 h, e.g., 16 h.

In another preferred embodiment, the step (a1) further includes subjecting to rotary evaporation in vacuum to remove the second solvent.

In another preferred embodiment, in the step (b1), the polymorphic transition in suspension comprises: after reacting for 16-24 h, transferring the solution sample to 0-10°C, and transferring the adherent material to a high-low temperature cycle (50~5°C) for further reaction for 3 days.

In another preferred embodiment, the method comprises:
(a2) mixing the compound of Formula A with a second solvent, then mixing with maleic acid to obtain maleate of the compound of Formula A; the second solvent is one or more selected from the group consisting of: 1,2-dichloroethane and methanol;
(b2) mixing the maleate of the compound of Formula A with a third solvent, subjecting to polymorphic transition in suspension, and crystallizing out to obtain maleate crystalline form I of the compound of Formula A; the third solvent is acetone.

In another preferred embodiment, the molar ratio of the compound of Formula A to maleic acid is (0.8-1.2):(0.8-1.2), preferably (0.9-1.1):(0.9-1.1), more preferably (0.95-1.05):(0.95-1.05), e.g., 1:1.05, 1:1.

In another preferred embodiment, the step (a2) is performed at 0-10°C, e.g., 5°C.

In another preferred embodiment, the salt-forming reaction time of the step (a2) is 8-20 h, e.g., 16 h.

In another preferred embodiment, in the step (b2), the polymorphic transition in suspension comprises: after reacting at room temperature for 12-20 h, filtering the resulting suspension.

In another preferred embodiment, the method comprises:
(a3) mixing the compound of Formula A with a second solvent, then mixing with maleic acid to obtain maleate of the compound of Formula A; the second solvent is methanol;
(b3) mixing the maleate of the compound of Formula A with a third solvent, subjecting to polymorphic transition in suspension, and crystallizing out to obtain maleate crystalline form II of the compound of Formula A; the third solvent is methyl *tert-butyl* ether.

In another preferred embodiment, the molar ratio of the compound of Formula A to maleic acid is (0.8-1.2):(0.8-1.2), preferably (0.9-1.1):(0.9-1.1), more preferably (0.95-1.05):(0.95-1.05), e.g., 1:1.05, 1:1.

In another preferred embodiment, the step (a2) is performed at room temperature, e.g., 15-40°C.

In another preferred embodiment, the salt-forming reaction time of the step (a2) is 1-6 h, e.g., 4 h.

In another preferred embodiment, in the step (b2), the polymorphic transition in suspension comprises: after reacting for 12-20 h, filtering the resulting suspension.

In another preferred embodiment, the method comprises:
(a4) mixing the compound of Formula A with a second solvent, then mixing with oxalic acid to obtain oxalate of the compound of Formula A; the second solvent is one or more of 1,2-dichloroethane and methanol;
(b4) mixing the oxalate of the compound of Formula A with a third solvent, subjecting to polymorphic transition in suspension, and crystallizing out to obtain oxalate crystalline form I of the compound of Formula A; the third solvent is acetone.

In another preferred embodiment, the molar ratio of the compound of Formula A to oxalic acid is (0.8-1.2):(0.8-1.2), preferably (0.9-1.1):(0.9-1.1), more preferably (0.95-1.05):(0.95-1.05), e.g., 1:1.05, 1:1.

In another preferred embodiment, the step (a4) is performed at 0-10°C, e.g., 5°C.

In another preferred embodiment, the salt-forming reaction time of the step (a4) is 8-20 h, e.g., 16 h.

In another preferred embodiment, in the step (b4), the polymorphic transition in suspension comprises: after reacting for 20 h, filtering the resulting suspension, transferring the solution sample to 5°C, and transferring the adherent material to a high-low temperature cycle (50~5°C) for further reaction.

In another preferred embodiment, the method comprises:
(c5) heating the oxalate crystalline form I of the compound of Formula A to a certain temperature, performing polymorphic transition to obtain oxalate crystalline form II.

In another preferred embodiment, heating the oxalate crystalline form I of the compound of Formula A to 180-200°C, performing polymorphic transition to obtain oxalate crystalline form II.

In another preferred embodiment, the method comprises:
(a6) mixing the compound of Formula A with a second solvent, then mixing with methanesulfonic acid to obtain mesylate of the compound of Formula A; the second solvent is one or more selected from the group consisting of 1,2-dichloroethane and methanol;
(b6) mixing the mesylate of the compound of Formula A with a third solvent, subjecting to polymorphic transition in suspension, and crystallizing out to obtain mesylate crystalline form I of the compound of Formula A; the third solvent is acetone.

In another preferred embodiment, the molar ratio of the compound of Formula A to methanesulfonic acid is (0.8-1.2):(0.8-1.2), preferably (0.9-1.1):(0.9-1.1), more preferably (0.95-1.05):(0.95-1.05), e.g., 1:1.05, 1:1.

In another preferred embodiment, the step (a6) is performed at 0-10°C, e.g., 5°C.

In another preferred embodiment, the salt-forming reaction time of the step (a6) is 8-20 h, e.g., 16 h.

In another preferred embodiment, the step (a6) further comprises: after completion of the reaction, subjecting to rotary evaporation in vacuum to remove solvent.

In another preferred embodiment, in step (b6), the polymorphic transition in suspension comprises: after reacting at room temperature for 20 h, filtering the resulting suspension.

In another preferred embodiment, the method comprises:
(a7) mixing the compound of Formula A with a second solvent, then mixing with methanesulfonic acid to obtain mesylate of the compound of Formula A; the second solvent is methanol;
(b7) mixing the mesylate of the compound of Formula A with a third solvent, subjecting to polymorphic transition in suspension, and crystallizing out to obtain mesylate crystalline form II of the compound of Formula A; the third solvent is methyl *tert-butyl* ether.

In another preferred embodiment, the molar ratio of the compound of Formula A to methanesulfonic acid is (0.8-1.2):(0.8-1.2), preferably (0.9-1.1):(0.9-1.1), more preferably (0.95-1.05):(0.95-1.05), e.g., 1:1.05, 1:1.

In another preferred embodiment, the step (a7) is performed at room temperature, e.g., 15-40°C.

In another preferred embodiment, the salt-forming reaction time of the step (a7) is 1-6 h, e.g., 4 h.

In another preferred embodiment, in the step (b7), the polymorphic transition in suspension comprises: after reacting for 18-36 h, filtering the resulting suspension.

In the third aspect of the present invention, provided is a pharmaceutical composition comprising an acid salt crystalline form of the compound of Formula A as described in the first aspect of the present invention, and pharmaceutically acceptable carriers.

In the fourth aspect of the present invention, provided is use of the acid salt crystalline form of the compound of Formula A as described in the first aspect of the present invention for the manufacture of a medicament, wherein the medicament is used for the uses selected from the group consisting of:
1) treating diseases or disorders associated with modulation of EGFR and/or HER2;
2) treating tumors; and
3) inhibiting cell proliferation.

In another preferred embodiment, the disease or disorder associated with modulation of EGFR and/or HER2 comprises one or more of tumor, sarcoma, and pain.

In another preferred embodiment, the cell is selected from the group consisting of: breast cancer cell, cervical cancer cell, colon cancer cell, lung cancer cell, gastric cancer cell, rectal cancer cell, pancreatic cancer cell, brain cancer cell, skin cancer cell, oral cancer cell, prostate cancer cell, bone cancer cell, renal cancer cell, ovarian cancer cell, bladder cancer cell, liver cancer cell, fallopian tube tumor cell, peritoneal tumor cell, melanoma cell, glioma cell, neuroglioblastoma cells, head and neck cancer cell, papillary renal tumor cell, leukemia cell, lymphoma cell, myeloma cell, and thyroid tumor cell.

In another preferred embodiment, the tumor is one or more of breast cancer, cervical cancer, colon cancer, lung cancer, gastric cancer, rectal cancer, pancreatic cancer, brain cancer, skin cancer, oral cancer, prostate cancer, bone cancer, renal cancer, ovarian cancer, bladder cancer, liver cancer, fallopian tube tumor, peritoneal tumor, melanoma, glioma, neuroglioblastoma, head and neck cancer, papillary renal tumor, leukemia, lymphoma, myeloma, and thyroid tumor.

It should be understood that within the scope of the present invention, the above-described technical features of the present invention and the technical features described in detail below (e.g., examples) may be combined with each other to constitute a new or preferred technical solution. Limited by space, it will not be repeated here.

### DESCRIPTION OF THE DRAWINGS

Figure 1 is the X-ray powder diffraction pattern of citrate crystalline form I of the present invention.
Figure 2 is the DSC-TGA pattern of citrate crystalline form I of the present invention.
Figure 3 is the X-ray powder diffraction pattern of free base crystalline form I of the present invention.
Figure 4 is the PLM image of free base crystalline form I of the present invention.
Figure 5 is the DSC-TGA pattern of free base crystalline form I of the present invention.
Figure 6 is the X-ray powder diffraction pattern of maleate crystalline form I of the present invention.
Figure 7 is the DSC-TGA pattern of maleate crystalline form I of the present invention.
Figure 8 is the X-ray powder diffraction pattern of maleate crystalline form II of the present invention.
Figure 9 is the DSC-TGA pattern of maleate crystalline form II of the present invention.
Figure 10 is the X-ray powder diffraction pattern of oxalate crystalline form I of the present invention.
Figure 11 is the DSC-TGA pattern of oxalate crystalline form I of the present invention.
Figure 12 is the X-ray powder diffraction pattern of oxalate crystalline form II of the present invention.
Figure 13 is the DSC-TGA pattern of oxalate crystalline form II of the present invention.
Figure 14 is the X-ray powder diffraction pattern of mesylate crystalline form I of the present invention.
Figure 15 is the DSC-TGA pattern of mesylate crystalline form I of the present invention.
Figure 16 is the X-ray powder diffraction pattern of mesylate crystalline form II of the present invention.
Figure 17 is the DSC-TGA pattern of mesylate crystalline form II of the present invention.
Figure 18 is the single-crystal structure of citrate crystalline form I of the present invention.

### DETAILED DESCRIPTION OF THE INVENTION

After long-term and in-depth research, the present inventors have prepared a series of acid salt crystalline forms of the compound of Formula A with excellent stability, especially citrate crystalline form I. While the crystalline forms of the present invention exhibit excellent stability, they also show better pharmacokinetics and higher *in vivo* exposure compared with the free base crystalline form. On this basis, the inventors have completed the present invention.

### Crystalline Form

A crystalline form is the solid substance state of a drug. Research on drug crystalline forms is research on the fundamental state of a drug. Only with a sufficiently full and comprehensive understanding of the crystalline state of a chemical drug, is it possible to find a drug crystalline solid, which is more suitable for the treatment of diseases. Drug crystalline forms can affect the physicochemical properties of a drug, which directly affects the basis of a drug to exert its therapeutic effects clinically. Different crystalline forms of the same drug may differ significantly in appearance, solubility, melting point, dissolution rate, bioavailability, etc., thereby affecting the stability, bioavailability, and efficacy of the drug. Therefore, the study of stable crystalline forms of the compound is of great significance.

The active ingredient of the same drug generally exists in two or more crystalline forms, known as drug polymorphism. Different crystalline forms have different solubility and dissolution rates, which affect the clinical therapeutic effect of the drug by causing changes in *in vivo* bioavailability. Different drug crystalline forms may affect dissolution and absorption *in vivo,* thereby influencing the bioavailability, clinical efficacy, and safety of the drug. Meanwhile, the stability of drug crystalline forms is also very important. To improve the bioavailability of a drug, reduce toxicity, and enhance therapeutic effects, greater attention should be paid to the stability of drug crystalline forms. Crystalline forms with good stability can ensure the physicochemical stability of pharmaceutical dosage forms during preparation and storage, maintain good solubility and bioavailability of pharmaceutical dosage forms, and guarantee equivalence between batches of the drug. The same drug often has multiple crystalline forms. To date, the crystalline form with better therapeutic effect and the most suitable for clinical application is called the advantageous drug crystalline form.

In the present invention, the acid salt crystalline forms of the compound represented by Formula (A) were screened to identify as many different crystalline forms as possible. The screened crystalline forms were characterized by X-ray powder diffraction (XRPD), differential scanning calorimetry (DSC), thermogravimetric analysis (TGA), liquid-state proton nuclear magnetic resonance (¹H NMR), and high-performance liquid chromatography (HPLC), and further researched and investigated by thermodynamic stability and hygroscopicity, to determine the advantageous drug crystalline form - citrate crystalline form I, thereby providing a reference for crystalline form selection for subsequent pharmacokinetic and animal studies.

Further pharmacokinetic studies show that citrate crystalline form I of the compound represented by Formula (A) provided by the present invention is easily absorbed in organisms and has excellent stability, excellent *in vivo* plasma concentration distribution, and high bioavailability. Meanwhile, due to its excellent physical stability, it has a longer shelf life and is highly conducive to drug quality control in later formulation development, enabling the drug to achieve better therapeutic effects.

In the present invention, provided is a citrate crystalline form I of the compound of Formula A, the X-ray powder diffraction pattern of the citrate crystalline form I comprises characteristic peaks at the following 20 values: 6.9±0.2°, 7.9±0.2°, 13.3±0.2°, 16.0±0.2°, and 18.6±0.2°.

In another preferred embodiment, the X-ray powder diffraction pattern of the citrate crystalline form I comprises characteristic peaks at the following 20 values: 6.9±0.2°, 7.9±0.2°, 11.4±0.2°, 13.3±0.2°, 15.7±0.2°, 16.0±0.2°, 16.7±0.2°, 18.6±0.2°, and 19.2±0.2°, 20.8±0.2°.

In another preferred embodiment, the X-ray powder diffraction pattern of the citrate crystalline form I comprises characteristic peaks at the following 20 values: 3.5±0.2°, 6.9±0.2°, 7.4±0.2°, 7.9±0.2°, 10.0±0.2°, 10.3±0.2°, 10.7±0.2°, 11.4±0.2°, 13.3±0.2°, 13.9±0.2°, 14.9±0.2°, 15.7±0.2°, 16.0±0.2°, 16.7±0.2°, 17.2±0.2°, 17.8±0.2°, 18.6±0.2°, 19.2±0.2°, 20.8±0.2°, 21.0±0.2°, 21.4±0.2°, 21.7±0.2°, 22.9±0.2°, 23.8±0.2°, 24.8±0.2°, 25.8±0.2°, 27.1±0.2°, 27.3±0.2°, 27.6±0.2°, and 28.5±0.2°.

In another preferred embodiment, the X-ray powder diffraction pattern of the citrate crystalline form I is substantially as shown in Figure 1.

In another preferred embodiment, the citrate crystalline form I exhibits a thermogravimetric analysis (TGA) spectrum and a differential scanning calorimetry (DSC) spectrum as shown in Figure 2.

In another preferred embodiment, the citrate crystalline form I is triclinic system, space group P-1, the unit cell parameters are a = 11.9331(2) Å, b = 13.4684(2) Å, c = 13.6474(2) Å, α = 69.2800(10)°, β = 82.1490(10)°, γ = 85.5730(10)°, V = 2031.32(6) Å³, Z = 2.

In another preferred embodiment, the compound of Formula A further comprise the crystalline form selected from the group consisting of: maleate crystalline form I, maleate crystalline form II, oxalate crystalline form I, oxalate crystalline form II, and mesylate crystalline form II.

Wherein, the X-ray powder diffraction pattern of the maleate crystalline form I comprises characteristic peaks at the following 2θ values: 6.7±0.2°, 9.6±0.2°, 14.3±0.2°, 14.6±0.2°, and 23.5±0.2°;
the X-ray powder diffraction pattern of the maleate crystalline form II comprises characteristic peaks at the following 2θ values: 6.9±0.2°, 10.6±0.2°, 14.5±0.2°, 16.5±0.2°, and 17.0±0.2°;
the X-ray powder diffraction pattern of the oxalate crystalline form I comprises characteristic peaks at the following 2θ values: 9.4±0.2°, 12.0±0.2°, 12.7±0.2°, 13.7±0.2°, and 21.4±0.2°;
the X-ray powder diffraction pattern of the oxalate crystalline form II comprises characteristic peaks at the following 2θ values: 9.5±0.2°, 12.2±0.2°, 12.7±0.2°, 13.7±0.2°, and 24.8±0.2°; and
the X-ray powder diffraction pattern of the mesylate crystalline form II comprises characteristic peaks at the following 20 values: 7.3±0.2°, 9.9±0.2°, 10.9±0.2°, 13.1±0.2°, and 14.2±0.2°.

In another preferred embodiment, the X-ray powder diffraction pattern of the maleate crystalline form I comprises characteristic peaks at the following 20 values: 6.7±0.2°, 8.1±0.2°, 9.6±0.2°, 14.3±0.2°, 14.6±0.2°, 15.8±0.2°, 16.4±0.2°, 19.4±0.2°, 19.9±0.2°, 20.2±0.2°, 21.4±0.2°, 22.4±0.2°, and 23.5±0.2°.

The X-ray powder diffraction pattern of the maleate crystalline form II comprises characteristic peaks at the following 2θ values: 6.9±0.2°, 10.6±0.2°, 14.5±0.2°, 15.5±0.2°, 16.1±0.2°, 16.5±0.2°, 17.0±0.2°, 19.4±0.2°, 21.5±0.2°, and 22.0±0.2°.

The X-ray powder diffraction pattern of the oxalate crystalline form I comprises characteristic peaks at the following 2θ values: 6.8±0.2°, 9.0±0.2°, 9.4±0.2°, 10.6±0.2°, 12.0±0.2°, 12.7±0.2°, 13.7±0.2°, 14.0±0.2°, 14.2±0.2°, 15.3±0.2°, 19.9±0.2°, 20.6±0.2°, 21.4±0.2°, 22.5±0.2°, 23.6±0.2°, and 24.9±0.2°.

In another preferred embodiment, The X-ray powder diffraction pattern of the oxalate crystalline form II comprises characteristic peaks at the following 20 values: 6.8±0.2°, 9.1±0.2°, 9.5±0.2°, 10.6±0.2°, 12.2±0.2°, 12.7±0.2°, 13.7±0.2°, 13.8±0.2°, 14.3±0.2°, 14.5±0.2°, 15.2±0.2°, 17.2±0.2°, 19.3±0.2°, 19.9±0.2°, 21.1±0.2°, 21.9±0.2°, 23.1±0.2°, 23.8±0.2°, and 24.8±0.2°.

The X-ray powder diffraction pattern of the mesylate crystalline form I comprises characteristic peaks at the following 20 values: 5.4±0.2°, 8.8±0.2°, 10.2±0.2°, 11.0±0.2°, 15.6±0.2°, 16.7±0.2°, 17.0±0.2°, 17.6±0.2°, 18.5±0.2°, 19.2±0.2°, 19.6±0.2°, 20.3±0.2°, 21.5±0.2°, 22.1±0.2°, 23.4±0.2°, 25.0±0.2°, and 29.7±0.2°.

The X-ray powder diffraction pattern of the mesylate crystalline form II comprises characteristic peaks at the following 20 values: 7.2±0.2°, 7.3±0.2°, 8.8±0.2°, 9.4±0.2°, 9.9±0.2°, 10.9±0.2°, 12.5±0.2°, 13.1±0.2°, 14.2±0.2° , 14.9±0.2°, 16.6±0.2°, 16.9±0.2°, 17.3±0.2°, 18.1±0.2°, 19.3±0.2°, 19.5±0.2°, 19.9±0.2°, 21.4±0.2°, 22.1±0.2°, 22.6±0.2°, 23.4±0.2°, 24.2±0.2°, and 26.3±0.2°.

### Preparation Method

The present invention also provides a method for preparing an acid salt crystalline form of the compound of Formula A, and preferably provides a method for preparing citrate crystalline form I, characterized in that the method is selected from the group consisting of:
method 1: dissolving the free base compound of Formula A in an organic solvent, stirring, then adding citric acid, continuing stirring, and subjecting to solid-liquid separation to obtain the citrate crystalline form I; and
method 2: adding an organic solvent to the citrate of the compound of Formula A, subjecting to polymorphic transition in suspension, and subjecting to solid-liquid separation to obtain the citrate crystalline form I.

In another preferred embodiment, the method is selected from the group consisting of:
method 1: mixing the compound of Formula A with solvent m, adding citric acid, and crystallizing out; wherein the solvent m is one of methanol, acetone, and acetonitrile;
method 2: mixing the citrate of the compound of Formula A with solvent n, subjecting to polymorphic transition in suspension, and crystallizing out; wherein solvent n is one or a mixed solvent selected from dichloromethane, acetone, tetrahydrofuran, and acetonitrile.

In another preferred embodiment, the compound of Formula A is free base crystalline form I.

### Use

The present invention provides a use of an acid salt crystalline form of the compound of Formula A, and preferably provides a use of citrate crystalline form I in the manufacture of a medicament for the prevention and/or treatment of diseases pathologically mediated by EGFR and/or HER2.

In another preferred embodiment, the diseases pathologically mediated by EGFR and/or HER2 include cancer, sarcoma, and pain.

In another preferred embodiment, the cancer is one or more of breast cancer, cervical cancer, colon cancer, lung cancer, gastric cancer, rectal cancer, pancreatic cancer, brain cancer, skin cancer, oral cancer, prostate cancer, bone cancer, renal cancer, ovarian cancer, bladder cancer, liver cancer, fallopian tube tumor, peritoneal tumor, melanoma, glioma, neuroglioblastoma, head and neck cancer, papillary renal tumor, leukemia, lymphoma, myeloma, and thyroid tumor.

### Pharmaceutical Composition and Administration Method

The present invention also provides a pharmaceutical composition, comprising a therapeutically effective amount of one or more of the acid salt crystalline forms and a pharmaceutically acceptable carrier.

Since the acid salt crystalline forms of the present invention exhibit excellent EGFR/HER2 kinase inhibitory activity, the crystalline forms of the present invention can be used for the treatment, prevention, and alleviation of diseases associated with EGFR/HER2 kinase inhibition.

The pharmaceutical composition of the invention comprises the acid salt crystalline form of the present invention or the pharmaceutically acceptable salts thereof in a safe and effective dosage range, and pharmaceutically acceptable excipients or carriers. Wherein, "safe and effective amount" means that the amount of the crystalline form is sufficient to significantly improve the condition without causing serious side effects. Generally, the pharmaceutical composition contains 1-2000 mg of the crystalline form of the present invention/dose, more preferably, 10-1000 mg of the crystalline form of the present invention/dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with each other and with the crystalline form of the present invention without significantly reducing the efficacy of the crystalline form. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, powders, or granules.

There is no special limitations on the administration mode of the crystalline form or pharmaceutical compositions of the present invention, and representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrants such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or a mixture thereof. In capsules, tablets, and pills, the dosage form may also include a buffer.

Solid dosage forms such as tablets, sugar pills, capsules, pills, and granules can be prepared using coatings and shell materials, such as casings and other well-known materials in the art. They can contain opaque agents. The active compounds or compounds in the compositions can be released in a delayed manner in a given part of the digestive tract. Examples of embedding components that can be used are polymeric substances and waxes. If necessary, the active compound can also form microcapsules with one or more of the aforementioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition can also include additives such as wetting agents, emulsifiers and suspending agents, sweeteners, flavoring agents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols, and their suitable mixtures.

The dosage forms of the crystalline form of the present invention used for topical administration include ointments, powders, patches, sprays, and inhalants. The active ingredients are mixed under sterile conditions with physiologically acceptable carriers and any preservatives, buffers, or propellants if necessary.

The crystalline form of the present invention can be administered alone or in combination with other pharmaceutically acceptable compounds.

The treatment method of the present invention can be administered alone or in combination with other treatment means or therapeutic drugs.

When the pharmaceutical composition is used, a safe and effective amount of the crystalline form of the present invention is administered to a mammal (such as a human) in need of treatment, wherein the dosage at the time of administration is the pharmaceutically effective dosage, for people having a body weight of 60kg, the daily dose is usually 1 ~2000mg, preferably 50~ 1000mg. Of course, the specific dosage should also consider factors such as the route of administration and the patient's health status, which are within the skill range of a skilled physician.

**Compared with the prior art, the present invention has the following main advantages:**
1) The crystalline forms exhibit excellent stability and high solubility;
2) compared with the free base crystalline form I, the citrate crystalline form I of the present invention possesses superior pharmacokinetic properties and higher *in vivo* exposure in animals.

The present invention will be further explained below in combination with specific examples. It should be understood that these examples are only used to illustrate the present invention and not to limit the scope of the present invention. In the following examples, the experimental methods without specific conditions are generally performed according to conventional conditions or those recommended by the manufacturer. Unless otherwise specified, percentages and parts are calculated by weight.

Unless defined otherwise, all technical and scientific terms used herein have the same meaning as commonly understood by one of ordinary skill in the art. In addition, any methods and materials similar or equivalent to those described herein can be used in the methods of the present invention. The preferred implementation methods and materials described herein are for illustrative purposes only.

Abbreviations used in the present invention are as follows:
- DSC: Differential Scanning Calorimetry
- DVS: Dynamic Vapor Sorption
- HPLC: High Performance Liquid Chromatography
- NMR: Nuclear Magnetic Resonance spectroscopy
- PLM: Polarized Light Microscopy
- TGA: Thermogravimetric Analysis
- XRPD: X-ray Powder Diffraction
- Fasted-State Simulated Intestinal Fluid: FaSSIF
- Fed-State Simulated Intestinal Fluid: FeSSIF
- Simulated Gastric Fluid: Simulated Gastric Fluid

The technical solution of the present invention is described in detail below.

### 1. Synthesis of the compound of Formula (A)

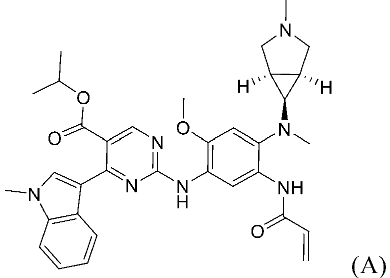

The detailed preparation procedure was the same as described in Patent Application PCT/CN2023/081557. The synthetic route was as follows:

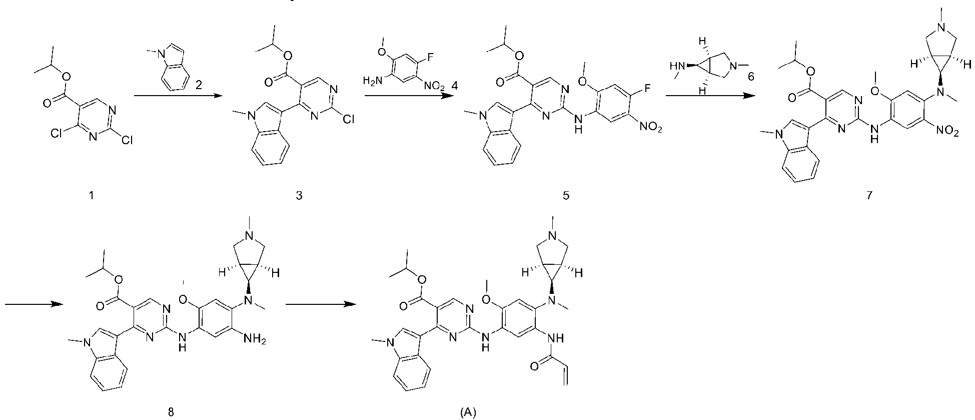

The obtained solid of Formula (A) was used to prepare the free base crystalline form according to the method provided in Patent Application PCT/CN2023/081557, and the free base crystalline form was used as the starting material for formation experiments of salt crystalline forms.

### 2. Preparation and characterization of acid salts of the compound of Formula (A)

Twelve acids used in the salt crystalline forms screening of the present invention are listed in Table 1.

**Table 1 Information on acids**

| No. | Name |
|---|---|
| 1 | Citric acid |
| 2 | Maleic acid |
| 3 | Methanesulfonic acid |
| 4 | Oxalic acid |
| 5 | Fumaric acid |
| 6 | L-Tartaric acid |
| 7 | Hydrochloric acid |
| 8 | L-Malic acid |
| 9 | Acetic acid |
| 10 | Benzoic acid |
| 11 | Phosphoric acid |
| 12 | Succinic acid |

Information on the solvents used in the salt crystalline forms screening of the present invention is shown in Table 2.

**Table 2 Solvent information**

| No. | Solvent | English/Abbr. | No. | Solvent | English/Abbr. |
|---|---|---|---|---|---|
| 1 | Dichloromethane | DCM | 11 | Butanone | MEK |
| 2 | 1,2-Dichloroethane | DCE | 12 | Methyl *tert-butyl* ether | MTBE |
| 3 | N-Methylpyrrolidone | NMP | 13 | Tetrahydrofuran | THF |
| 4 | Dimethyl sulfoxide | DMSO | 14 | 1,4-Dioxane | 1,4-dioxane |
| 5 | Methanol | MeOH | 15 | Acetonitrile | ACN |
| 6 | Ethanol | EtOH | 16 | Water | H₂O |
| 7 | Isopropanol | IPA | 17 | n-Heptane | Heptane/ Hept. |
| 8 | Ethyl acetate | EtOAc | 18 | Cyclohexane | Cyclohexane |
| 9 | Isopropyl acetate | IPAC | 19 | Diisopropyl ether | IPE |
| 10 | Acetone | Acetone | N/A | | |

### 2.1 Characterization of starting material

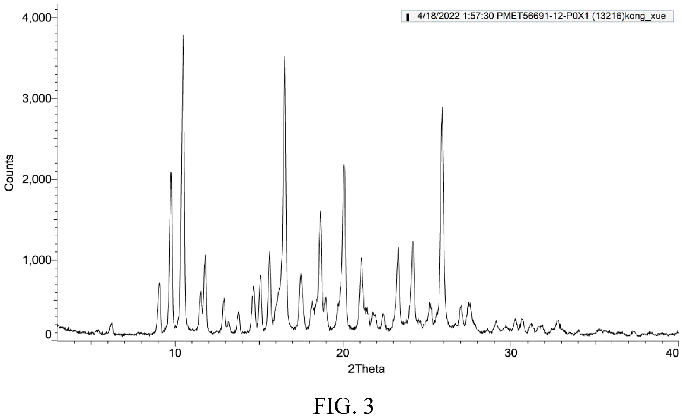

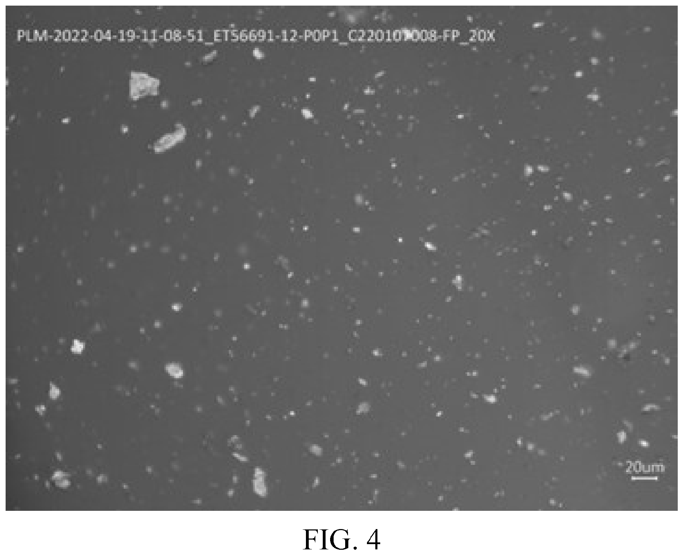

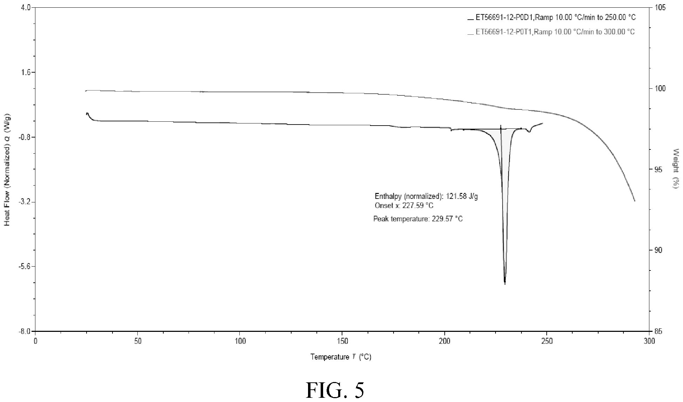

Comprehensive characterization was performed on the prepared free base crystalline form I. XRPD (Figure 3) and PLM (Figure 4) indicate that the free base crystalline form is crystals with good crystallinity and irregular morphology. DSC (Figure 5) curve shows an endothermic peak at 230 °C, assigned to the melting point. TGA curve shows no significant weight loss before decomposition. ¹H-NMR detected solvent residues of 0.85% THF and 0.09% heptane. The results are summarized in Table 3 below.

**Table 3 Characterization results of the starting material**

| Item | Test Result |
|---|---|
| XRPD | Crystals with good crystallinity, free base crystalline form I |
| PLM | Irregular-shaped crystals |
| DSC, endo Onset/peak(°C), ΔH(J/g) | 228/230 °C (122) |
| **TGA (weight** loss % /@ temperature) | No significant weight loss before decomposition |
| ¹H-NMR | 0.85% **THF** + 0.09% Heptane |

### 2.2 Visual solubility

At room temperature, the solubility of free base crystalline form I was roughly determined by visual inspection in 18 solvents. The results are shown in Table 4. Except for good solubility in DCM and DCE, the solubility was poor in most tested solvents.

**Table 4 Results of visual solubility**

| No. | Solvent | Solubility (mg/mL) | No. | Solvent | Solubility (mg/mL) |
|---|---|---|---|---|---|
| 1 | DCM | > 107 | 10 | MeOH | < 0.40 |
| 2 | DCE | 61.75-123.50 | 11 | MTBE | < 0.33 |
| 3 | NMP | 18.25-27.38 | 12 | Heptane | < 0.30 |
| 4 | Acetone | 2.22-4.44 | 13 | 1,4-dioxane | < 0.27 |
| 5 | DMSO | 2.60-2.93 | 14 | IPAC | < 0.30 |
| 6 | MEK | 2.78-4.44 | 15 | Cyclohexane | < 0.33 |
| 7 | THF | 2.55-3.19 | 16 | H₂O | < 0.27 |
| 8 | ACN | 0.93-1.01 | 17 | IPA | < 0.35 |
| 9 | EtOH | < 0.30 | 18 | EtOAc | < 0.29 |

### 2.3 Preparation of salt crystalline form

### 2.3.1 Citrate crystalline form I

50 mg of free base crystalline form I was weighed and put into a vial and dissolved in 0.5 mL of DCE at room temperature, followed by addition of a methanol solution of citric acid (0.1 M, 1.05 eq.). After the mixture was reacted at 5 °C for 16 h, the solvent was removed by rotary evaporation in vacuum. 1 mL of acetone was added to the concentrated product, and the suspension was subjected to polymorphic transition at room temperature. After reacted for 20 h, the solution sample was transferred to 5 °C, and the sticky material was subjected to high-low temperature cycling (50~5 °C) for an additional 3 days. The solid obtained after filtration was dried under vacuum at 50 °C and characterized.

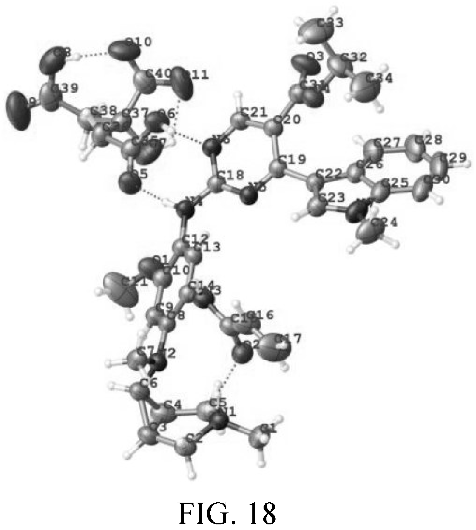

The DSC and TGA results of citrate crystalline Form I are shown in Figure 2. TGA shows a weight loss of 16.8% at 164-218 °C, attributed to decomposition. No obvious weight loss was observed before decomposition. NMR shows a solvent residue of acetone at 0.74%. Based on the above results, the obtained citrate is believed to be an anhydrous crystalline form. The citrate crystalline form I was subjected to single-crystal structure analysis, and the result is shown in Figure 18.

Its XRPD pattern is shown in Figure 1, and the data is listed in Table 5:

**Table 5**

| 2θ(°) | Relative Intensity | 2θ(°) | Relative Intensity |
|---|---|---|---|
| 3.5 | 3.1% | 21.7 | 6.3% |
| 6.9 | 100.0% | 21.9 | 1.8% |
| 7.4 | 14.5% | 22.9 | 17.6% |
| 7.9 | 54.3% | 23.3 | 4.5% |
| 9.6 | 2.0% | 23.8 | 14.5% |
| 10.0 | 7.6% | 24.3 | 4.2% |
| 10.3 | 2.1% | 24.8 | 7.5% |
| 10.7 | 3.3% | 25.1 | 2.4% |
| 11.4 | 25.4% | 25.8 | 9.5% |
| 13.3 | 80.6% | 26.2 | 4.1% |
| 13.9 | 9.1% | 26.6 | 3.4% |
| 14.9 | 18.9% | 27.1 | 13.7% |
| 15.7 | 31.0% | 27.3 | 6.2% |
| 16.0 | 41.6% | 27.6 | 7.2% |
| 16.7 | 22.9% | 28.1 | 2.6% |
| 17.2 | 18.5% | 28.5 | 6.6% |
| 17.8 | 8.0% | 29.1 | 1.8% |
| 18.6 | 43.5% | 30.0 | 2.3% |
| 19.2 | 28.6% | 30.6 | 3.8% |
| 19.7 | 4.1% | 34.9 | 2.0% |
| 20.8 | 24.6% | 36.9 | 3.0% |
| 21.0 | 16.5% | 37.8 | 2.4% |
| 21.4 | 9.4% | | |

### 2.3.2 Maleate crystalline form I

50 mg of the free base crystalline form was weighed and put into a vial and dissolved in 0.6 mL of 1,2-dichloroethane at room temperature, followed by addition of a freshly prepared methanol solution of maleic acid (0.1 M, 1.05 eq.). After the mixture was reacted at 5 °C for 16 h, the solvent was removed by rotary evaporation in vacuum. 1 mL of acetone was added to the concentrated product, and the suspension was subjected to polymorphic transition at room temperature. After reacted for 20 h, the resulting suspension was filtered. The filtered solid was dried under vacuum at 50 °C and characterized.

DSC and TGA results of maleate crystalline form I are shown in Figure 7. NMR result shows a solvent residue of acetone at 5.44%. TGA shows a weight loss of 5.5% at 72-152 °C. It is considered a solvate, designated as maleate crystalline form I.

Its XRPD pattern is shown in Figure 6, and the data is listed in Table 6:

**Table 6**

| 2θ(°) | Relative Intensity | 2θ(°) | Relative Intensity |
|---|---|---|---|
| 6.7 | 100.00% | 20.2 | 14.80% |
| 7.8 | 7.60% | 20.6 | 3.50% |
| 8.1 | 15.50% | 21.1 | 2.00% |
| 9.6 | 30.50% | 21.4 | 10.60% |
| 10.2 | 4.20% | 21.8 | 3.60% |
| 11.2 | 2.00% | 22.4 | 13.90% |
| 12.9 | 4.40% | 23.1 | 8.70% |
| 13.8 | 4.10% | 23.5 | 17.80% |
| 14.3 | 34.80% | 23.9 | 6.10% |
| 14.6 | 19.40% | 24.6 | 9.40% |
| 15.2 | 5.20% | 25.5 | 7.00% |
| 15.8 | 15.30% | 26.1 | 8.40% |
| 16.0 | 4.30% | 26.4 | 4.90% |
| 16.4 | 16.20% | 28.1 | 2.20% |
| 16.6 | 3.90% | 28.3 | 3.10% |
| 18.7 | 8.70% | 28.7 | 3.10% |
| 19.0 | 7.00% | 29.6 | 2.50% |
| 19.4 | 10.50% | 30.2 | 2.00% |
| 19.9 | 13.30% | 32.5 | 1.70% |
| | | 34.0 | 2.40% |

### 2.3.3 Maleate crystalline form II

50 mg of free base crystalline form I was weighed and put into a vial, and 0.2~0.5 mL of MeOH was added at room temperature (white suspension), followed by addition of a freshly prepared 0.1 M methanol solution of maleic acid (0.1 M, 1.05 eq.). After the mixture was reacted at room temperature for about 4 h, 6 mL of methyl *tert-butyl* ether was added. After the mixture was continued to react for 20 h, the suspension was filtered, dried under vacuum, and characterized.

The DSC and TGA results of maleate crystalline form II are shown in Figure 9. NMR result shows a solvent residue of MTBE at 0.37%. TGA shows a weight loss of 1.3% at 23.5-50 °C, corresponding to an endothermic peak in DSC at 47-71 °C. After heated to 120 °C by DSC and immediately tested, the crystalline form, DSC, and TGA of the heated sample remained unchanged, presuming that maleate crystalline form II may be a hydrate or a hygroscopic anhydrous form.

Its XRPD pattern is shown in Figure 8, and the data is listed in Table 7:

**Table 7**

| 2θ(°) | Relative Intensity | 2θ(°) | Relative Intensity |
|---|---|---|---|
| 6.9 | 100.0% | 19.1 | 4.0% |
| 7.3 | 3.2% | 19.4 | 10.6% |
| 8.7 | 1.2% | 20.0 | 1.8% |
| 9.8 | 6.5% | 20.2 | 3.3% |
| 10.6 | 22.5% | 21.1 | 5.7% |
| 11.1 | 1.1% | 21.5 | 9.8% |
| 11.6 | 3.1% | 22.0 | 10.9% |
| 13.0 | 1.3% | 22.4 | 1.4% |
| 14.2 | 3.2% | 22.8 | 2.0% |
| 14.5 | 15.7% | 23.1 | 8.3% |
| 15.0 | 2.4% | 24.2 | 6.4% |
| 15.5 | 8.4% | 25.2 | 6.9% |
| 16.1 | 11.3% | 25.8 | 6.8% |
| 16.5 | 23.7% | 26.8 | 3.6% |
| 17.0 | 16.1% | 27.3 | 6.6% |
| 17.3 | 3.4% | 28.5 | 1.4% |
| 17.7 | 1.6% | 29.5 | 0.8% |
| 18.1 | 2.2% | 30.1 | 2.4% |
| 18.6 | 3.6% | 32.4 | 1.0% |

### 2.3.4 Oxalate crystalline form I

50 mg of free base crystalline form I was weighed and put into a vial and dissolved in 0.6 mL of 1,2-dichloroethane at room temperature, followed by addition of a methanol or DCE solution of oxalic acid (1.05 eq.). After the mixture was reacted at 5 °C for 16 h, the solvent was removed by rotary evaporation in vacuum. 1 mL of acetone was added to the concentrated product, and the suspension was subjected to polymorphic transition at room temperature. After reacted for 20 h, the suspension was filtered. The solution sample was transferred to 5 °C, and the sticky material was subjected to high-low temperature cycling (50~5 °C) for further reaction. The filtered solid was dried under vacuum and characterized to obtain oxalate crystalline form I.

The DSC and TGA results of oxalate crystalline form I are shown in Figure 11. NMR indicates approximately 0.49 mol of acetone. TGA shows a corresponding weight loss of 4.9% at 100-200 °C. After oxalate crystalline form I was heated to 190 °C, its XRPD changed. Based on the above, oxalate crystalline form I is presumed to be an acetone solvate.

Its XRPD pattern is shown in Figure 10, and the data is listed in Table 8:

**Table 8**

| 2θ(°) | Relative Intensity | 2θ(°) | Relative Intensity |
|---|---|---|---|
| 6.8 | 36.9% | 22.5 | 37.3% |
| 9.0 | 28.8% | 22.9 | 25.3% |
| 9.4 | 67.9% | 23.2 | 24.0% |
| 10.6 | 28.7% | 23.6 | 32.7% |
| 12.0 | 100.0% | 24.3 | 11.9% |
| 12.7 | 62.7% | 24.9 | 47.4% |
| 13.7 | 50.3% | 25.9 | 9.1% |
| 14.0 | 38.1% | 26.2 | 7.9% |
| 14.2 | 31.6% | 26.4 | 12.2% |
| 15.3 | 31.3% | 26.7 | 10.3% |
| 15.6 | 10.5% | 27.4 | 7.5% |
| 16.9 | 11.5% | 27.6 | 6.2% |
| 17.6 | 12.4% | 28.3 | 9.5% |
| 18.2 | 15.3% | 28.8 | 3.2% |
| 18.6 | 12.7% | 29.1 | 4.2% |
| 19.1 | 28.5% | 29.6 | 3.7% |
| 19.5 | 8.2% | 30.9 | 8.0% |
| 19.9 | 45.4% | 32.1 | 4.2% |
| 20.6 | 46.9% | 32.5 | 3.1% |
| 21.1 | 8.6% | 34.0 | 3.0% |
| 21.4 | 56.2% | 36.2 | 3.1% |
| 22.1 | 8.9% | | |

### 2.3.5 Oxalate crystalline form II

The sample obtained after heating oxalate crystalline form I to 190 °C was characterized to give oxalate crystalline Form II. TGA result shows no significant weight loss before decomposition. The DSC and TGA results are shown in Figure 13. Thus, oxalate crystalline form II is likely an anhydrous crystalline form.

The XRPD pattern of oxalate crystalline form II is shown in Figure 12, and the data is listed in Table 9:

**Table 9**

| 2θ(°) | Relative Intensity | 2θ(°) | Relative Intensity |
|---|---|---|---|
| 6.8 | 22.3% | 19.3 | 20.9% |
| 7.0 | 6.1% | 19.9 | 42.7% |
| 9.1 | 38.0% | 21.1 | 24.2% |
| 9.5 | 100.0% | 21.9 | 29.4% |
| 10.6 | 38.7% | 22.2 | 11.8% |
| 11.5 | 8.8% | 23.1 | 26.0% |
| 12.2 | 64.2% | 23.8 | 23.3% |
| 12.7 | 55.4% | 24.8 | 53.9% |
| 13.7 | 43.9% | 25.8 | 12.8% |
| 13.8 | 23.3% | 26.7 | 14.3% |
| 14.3 | 37.3% | 27.0 | 13.1% |
| 14.5 | 42.0% | 27.6 | 8.5% |
| 15.2 | 20.0% | 27.9 | 4.2% |
| 15.5 | 17.3% | 28.8 | 8.1% |
| 17.2 | 20.3% | 29.3 | 4.9% |
| 18.0 | 11.7% | 29.9 | 8.1% |
| 18.7 | 8.5% | 31.3 | 7.1% |
| 19.1 | 19.4% | 33.4 | 3.7% |

### 2.3.6 Mesylate crystalline Form I

50 mg of free base crystalline form I was weighed and dissolved in 0.6 mL of DCE at room temperature, and a freshly prepared methanol solution of methanesulfonic acid (1.05 eq.) was added. After the mixture was reacted at 5 °C for 16 h, the solvent was removed by rotary evaporation in vacuum. 1 mL of acetone was added to the concentrated product, and the suspension was subjected to polymorphic transition at room temperature. After reacted for 20 h, the suspension was filtered, and the filtered solid was dried under vacuum at 50 °C for at least 3 h and characterized.

The DSC and TGA results of mesylate crystalline form I are shown in Figure 15. No obvious weight loss was observed before decomposition. DSC shows only one endothermic peak at 258 °C, assigned to melting peak. NMR shows a solvent residue of acetone at 0.33%. The salt formation ratio of free base to methanesulfonic acid is approximately 1:1, and the mesylate crystalline form I is believed to be an anhydrous crystalline form.

The XRPD pattern of mesylate crystalline form I is shown in Figure 14, and the data is listed in Table 10:

**Table 10**

| 2θ(°) | Relative Intensity | 2θ(°) | Relative Intensity |
|---|---|---|---|
| 5.4 | 15.7% | 20.3 | 27.7% |
| 8.8 | 52.0% | 21.5 | 27.1% |
| 9.6 | 3.6% | 22.1 | 88.8% |
| 10.2 | 34.7% | 22.7 | 27.2% |
| 11.0 | 79.7% | 23.4 | 100.0% |
| 12.1 | 9.4% | 23.9 | 9.7% |
| 12.9 | 10.8% | 25.0 | 64.2% |
| 13.8 | 10.5% | 25.8 | 12.5% |
| 14.2 | 10.5% | 26.3 | 5.5% |
| 14.8 | 6.1% | 27.8 | 3.8% |
| 15.6 | 52.0% | 28.6 | 9.2% |
| 16.7 | 93.8% | 29.0 | 7.9% |
| 17.0 | 34.6% | 29.7 | 18.6% |
| 17.6 | 27.8% | 30.5 | 8.5% |
| 17.9 | 14.9% | 31.1 | 10.1% |
| 18.5 | 47.4% | 31.8 | 7.3% |
| 19.2 | 86.8% | 37.0 | 3.7% |
| 19.6 | 26.4% | | |

### 2.3.7 Mesylate crystalline form II

50 mg of free base crystalline Form I was weighed and put into a vial, and 0.2 mL of MeOH was added at room temperature, followed by addition of a methanol solution of methanesulfonic acid (1.05 eq.). After the mixture was reacted at room temperature for about 4 h, 6 mL of methyl *tert-butyl* ether was added. After further reacted for 1 day, the suspension was filtered, dried at 50 °C under vacuum, and characterized.

The DSC and TGA results of mesylate crystalline form II are shown in Figure 17. A weight loss of 0.9% occurs at 23-80 °C, corresponding to an endothermic peak in DSC at 76 °C. NMR shows a residue of MTBE at only 0.18%. After mesylate crystalline form II was heated to 120 °C by DSC and immediately subjected to XRPD analysis, the crystalline form remained unchanged. The weight loss is believed to be attributed to surface-adsorbed water. Accordingly, mesylate crystalline form II is presumed to be an anhydrous crystalline form. Mesylate crystalline form II exhibits a set of polymorphic transition peaks at 168-189 °C and converts to mesylate crystalline form I upon heating to 210 °C. The endothermic peak of mesylate crystalline form II at 257 °C corresponds to the melting point of form I formed upon heating and polymorphic transition.

The XRPD pattern of mesylate crystalline form II is shown in Figure 16, and the data is listed in Table 11.

**Table 11**

| 2θ(°) | Relative Intensity | 2θ(°) | Relative Intensity |
|---|---|---|---|
| 3.3 | 11.5% | 18.9 | 7.1% |
| 7.2 | 32.5% | 19.3 | 20.1% |
| 7.3 | 100.0% | 19.5 | 15.5% |
| 8.1 | 7.2% | 19.9 | 18.5% |
| 8.8 | 37.6% | 20.6 | 9.1% |
| 9.4 | 20.3% | 21.0 | 8.9% |
| 9.9 | 38.1% | 21.4 | 23.2% |
| 10.9 | 88.5% | 22.1 | 30.0% |
| 12.5 | 28.1% | 22.6 | 26.7% |
| 13.1 | 66.1% | 23.4 | 28.4% |
| 14.2 | 75.9% | 24.2 | 16.8% |
| 14.9 | 23.7% | 25.0 | 6.0% |
| 15.5 | 12.6% | 25.9 | 10.6% |
| 16.3 | 4.6% | 26.3 | 17.6% |
| 16.6 | 35.6% | 27.0 | 5.0% |
| 16.9 | 30.0% | 27.7 | 5.3% |
| 17.3 | 22.8% | 28.7 | 3.7% |
| 18.1 | 19.7% | 29.7 | 3.4% |
| | | 30.1 | 3.1% |

The present invention employed methods including salt formation followed by polymorphic transition in suspension, and direct salt formation, and attempted to prepare salts from free base crystalline form I with various acids in different solvent systems. The characterization results of salts formed with phosphoric acid, fumaric acid, L-malic acid, hydrochloric acid, methanesulfonic acid, and other acids are summarized in Table 12.

**Table 12**

| Salt | Salt formation ratio (FB/Acid) | DSC, endo Onset/peak (°C), ΔH(J/g) | TGA Wt loss%/@T (°C) | ¹H NMR | Remarks |
|---|---|---|---|---|---|
| Phosphate | 1:0.94 | 41/78 (84); 134/140 (2); 174/178 (2) | 2.9%/24-100 °C; | 0.78% MeOH | Both did not exhibit a well-defined melting point |
| | / | 39/79 (88); 132/139 (3); | 2.1%/24-85 °C; 0.9%/85-160 °C | 1.91% MeOH | |
| Fumarate | ~1:0.92 | 40/92 (123); 133/159 (21); | 6.7%/25-190 °C; | 1.34% acetone | No obvious melting point observed |
| | ~1:0.72 | 38/83 (104); | 1.7%/24-65 °C; 6.3%/65-140 °C; | 5.81% (~0.51 mol) MTBE | |
| L-Malate | 1:0.96 | 127/137 (29); 147/152 (15); | 3.0%/75-130 °C; 1.7%/130-160 °C | 6.40% (~0.85 mol) acetone | Both are solvates |
| | 1:1.09 | ~25/61 (16); 123/137 (hump, 84); | 1.1%/23-80 °C; 7.4%/80-160 °C; | 6.96% (~0.61 mol) MTBE | |
| Hydrochloride | 1:0.84 | 86/101 (38); 172/184 (hump, 52); | 2.5%/ 30-96 °C; | 0.24% Acetone | Severe polymorphism, good hydrochloride crystals not obtained |
| | 1:1.61 | 36/79 (97); 147/163 (46); | 2.0%/ 40-110 °C; 7.2%/ 111-180 °C; | 1.14% Acetone | |
| | - | 43/76 (31); 169/176 (35); | 2.0%/ 22-50 °C; 2.9%/ 50-185 °C; | 0.90%IPA | |
| | - | 40/74 (32); 156/174 (62); | 2.0%/ 23-60 °C; 9.4%/ 60-175 °C; | 5.09% (~0.58 mol) IPA+0.89% MTBE | |
| | 1:1.12 | 84/93 (4); 108/133 (23); 176/189 (hump, 42) | 3.6%/ 23.5-100 °C; 1.5%/ 100-195 °C; | 0.45% MTBE | |
| | 1:1.67 | 46/85 (124); | 4.8%/ 22-90 °C; 5.3%/ 90-175 °C; | ~0.30% MTBE | |
| | 1:1.61 | 44/81 (56); 169/184 (45); | 3.7%/ 23-85 °C; 5.8%/ 85-160 °C; | 1.9%EtOAc | |
| | 1:1.07 | 87/105 (23); 170/181 (hump, 44) | 2.2%/ 25-70 °C; 2.2%/ 70-200 °C; | 0.17% acetic acid+0.26% EtOAc | |
| | 1:1.90 | 36/77 (98); 140/158 (87); | 2.3%/ 25-70 °C; 8.5%/ 70-180 °C; | 4.57% acetic acid+2.13% EtOAc | |
| | 1:1.07 | 88/94 (1); 108/137 (18); 170/184 (37) | 2.5%/ 24-100 °C; 2.4%/ 100-190 °C; | 0.18%ACN+2.10% IPE | |
| Trimethane-sulfonate | 1: 3.22 | 49/84 (187); 143/147(4); 185/196 (25) | 5.5%/24-68 °C | No obvious solvent residue | Possibly a hydrate |

### 3. Analytical Methods

### 3.1 X-ray Powder Diffraction (XRPD)

XRPD patterns were collected on a Bruker D2 Phaser. The sample was placed on a smooth, background-free silicon wafer for measurement. Measurement parameters are shown in Table 13.

**Table 13 XRPD Parameters**

| | | | |
|---|---|---|---|
| **Instrument** | Bruker, D2 Phaser | **Method name** | 3-40+0.02+0.2+15.bsml |
| **Radiation wavelength** | Cu Kα (λ = 1.5418 Å) | **Primary axial Soller** | 2.5° |
| **Detection mode** | Step scan | **Secondary axial Soller** | 2.5° |
| **Scan axis** | θs- θd | **Detector slit** | 5.827° |
| **Scan range** | 3-40° (2θ) | **Divergence slit width** | 0.6 mm |
| **Step size** | 50 mL/min | **Anti-scattering slit** | 0 mm |
| **Scan speed** | 0.02° (2θ) | **Rotation** | On |
| **Tube voltage/current** | 30 KV/10 mA | **Sample holder** | Zero background |

### 3.2 Polarized Light Microscopy (PLM)

PLM analysis was performed using an CNOPTEC BK-Pol microscope. A small amount of sample was taken and placed on a glass slide, dispersed in silicone oil, covered with a coverslip, and observed under the microscope.

### 3.3 Differential Scanning Calorimetry (DSC)

DSC curves were collected on a TA Instrument DSC 250. The test method of DSC 250 instrument is: an appropriate amount of sample was accurately weighed into a pierced aluminum crucible and heated from 25 °C to the final temperature at 10 °C/min. Instrument parameters are shown in Table 14.

**Table 14 DSC Parameters**

| | |
|---|---|
| **Instrument** | TA, 250 |
| **Sample pan** | Pierced aluminum crucible |
| **Temperature range** | 25 -300 °C |
| **Heating rate** | 10 °C/min |
| **Purge gas** | Nitrogen |
| **Flow rate** | 50 mL/min |

### 3.4 Thermogravimetric Analysis (TGA)

TGA data were collected on a TA Instrument TGA 550. An appropriate amount of sample was taken and placed in a pre-tared aluminum crucible and heated from room temperature to 300 °C at 10 °C/min. Temperature program and instrument parameters of TGA 550 are shown in Table 15.

**Table 15 TGA Parameters**

| | |
|---|---|
| **Instrument** | TA, 550 |
| **Sample pan** | Open aluminum crucible |
| **Temperature range** | RT - 300 °C |
| **Heating rate** | 10 °C/min |
| **Purge gas** | N₂ |
| **Flow rate** | Balance chamber: 40 mL/min |
| | Sample chamber: 25 mL/min |

### 3.5 Dynamic Vapor Sorption (DVS)

Moisture sorption/desorption data were collected on a DVS Intrinsic. Approximately 20-30 mg of sample was placed in a pre-tared sample chamber and automatically weighed. The sample was dried at 40 °C/0% RH until dm/dt ≤ 0.002%. After cooling to 25 °C and dm/dt ≤ 0.002%, test was performed using the parameters in Table 16.

**Table 16 DVS Parameters**

| | |
|---|---|
| **Instrument** | SMS, DVS Intrinsic |
| **Step time** | 60 min |
| **Drying/measuring temperature** | 40 °C/25 °C |
| **Cycle** | Full cycle |
| **Equilibration time per 10 RH** | 1 hour |
| **Saving frequency** | 5 s |
| **Total flow rate** | 200 sccm |
| **Total flow rate (post-test)** | 200 sccm |
| **Method for anhydrous** substance | Sorption: 0, 10, 20, 30, 40, 50, 60, 70, 80, 90 |
| | Desorption:80, 70, 60, 50, 40, 30, 20, 10, 0 |
| **Method for hydrate** | Sorption 1: 40, 50, 60, 70, 80, 90 |
| | Desorption 1: 80, 70, 60, 50, 40, 30, 20, 10, 0 |
| | Sorption 2: 0, 10, 20, 30, 40, 50, 60, 70, 80, 90 |
| | Desorption 2: 80, 70, 60, 50, 40, 30, 20, 10, 0 |

### 3.6 ¹H Nuclear Magnetic Resonance (¹H-NMR)

¹H-NMR data were collected on a Varian or Bruker 400 MHz spectrometer. Unless otherwise specified, samples were prepared in DMSO-d₆ and measured using the parameters in Table 17.

**Table 17 ¹H-NMR Parameters**

| | |
|---|---|
| **Instrument** | Varian or Bruker |
| **Frequency** | 400 mHz |
| **number of scans** | 8 |
| **Temperature** | ~25 °C |

### 3.7 High Performance Liquid Chromatography (HPLC)

HPLC analysis was performed on a Shimadzu SPD-20 system. The HPLC method for solubility and stability is shown in Table 18.

**Table 18 HPLC Method**

| | | | |
|---|---|---|---|
| **Instrument** | Shimadzu SPD-20 | | |
| **Column** | Agilent Eclipse Plus C18, 4.6 mm×250 mm, 5.0 µm | | |
| **Column temperature** | 30 °C | | |
| **Mobile phase** | MPA: 0.1% TFA in H₂O (v/v) | | |
| | MPB: ACN | | |
| **Flow rate** | 1.0 mL/min | | |
| **Injection volume** | 5 µL | | |
| **Detector &wavelength** | 220; 254 nm | | |
| **Run time** | 18 min | | |

| | Time (min) | A% | B% |
|---|---|---|---|
| **Gradient** | 0 | 90 | 10 |
| | 10 | 15 | 85 |
| | 15 | 15 | 85 |
| | 15.1 | 90 | 10 |
| | 18 | 90 | 10 |
| **Diluent & needle wash solution** | ACN/ H₂O(v/v): 1/1 | | |

### 4. Evaluation of acid salt crystalline forms and free base crystalline form of the compound of Formula (A)

In this salt screening study, 7 crystalline forms were obtained: citrate crystalline form I, maleate crystalline form I, maleate crystalline form II, oxalate crystalline form I, oxalate crystalline form II, mesylate crystalline form I, and mesylate crystalline form II.

Based on solid-state characterization of the salts and polymorph screening results of free phase, citrate crystalline form I, maleate crystalline form II, and mesylate crystalline form I were selected for solubility, stability, and pharmacokinetic studies.

### 4.1 Solubility Study

Solubility of citrate crystalline form I, maleate crystalline form II, mesylate crystalline form I, and free base crystalline form I was tested in biological medium and water at 37 °C. All results are shown in Table 19.

**Table 19 Results of solubility in biological medium and water**

| **Compound** | **Medium** | **0.5 h** | **2 h** | **24 h** |
|---|---|---|---|---|
| | | **Solubility (mg/mL)** | **Solubility (mg/mL)** | **Solubility (mg/mL)** |
| Citrate crystalline form I | SGF | > 5.04 | > 5.04 | > 5.04 |
| | FeSSIF | > 5.02 | > 5.02 | > 5.02 |
| | FaSSIF | >5.00 | > 5.00 | 3.99 |
| | H₂O | 1.91 | 1.99 | 2.44 |
| Maleate crystalline form II | SGF | > 5.01 | > 5.01 | > 5.01 |
| | FeSSIF | > 5.01 | > 5.01 | > 5.01 |
| | FaSSIF | > 5.03 | > 5.03 | 3.45 |
| | H₂O | 1.64 | 1.62 | 1.84 |
| Mesylate crystalline form I | SGF | >5.00 | > 5.00 | > 5.00 |
| | FeSSIF | >5.01 | > 5.01 | > 5.01 |
| | FaSSIF | >5.00 | > 5.00 | 3.41 |
| | H₂O | >5.00 | > 5.00 | > 5.00 |
| Free base crystalline form I | SGF | >5.00 | > 5.00 | > 5.00 |
| | FeSSIF | 3.53 | > 5.02 | > 5.02 |
| | FaSSIF | 0.13 | 0.13 | 0.18 |
| | H₂O | 0.01 | 0.01 | 0.01 |

The three salt crystalline forms (citrate crystalline form I, maleate crystalline form II, and mesylate crystalline form I) exhibited higher solubility than that of free base crystalline form I in water and biological medium (FaSSIF, FeSSIF), and comparable solubility in SGF.

### 4.2 Solid-State Stability Study

Appropriate amounts of citrate crystalline form I, maleate crystalline form II, and mesylate crystalline form I were taken respectively and spread open in vials and stored under five conditions for 7 days: 80 °C (3 d), 60 °C, 25 °C/60% RH, 40 °C/75% RH, and 25 °C/90±5% RH. Samples were taken at day 3 and day 7 for HPLC and XRPD analysis.

Stability studies showed that the crystalline form and chemical purity of citrate crystalline form I did not change significantly under the above five conditions; maleate crystalline form II transformed into an unstable hydrate of maleate after 7 days under the condition of 25 °C /90±5%RH, while no significant changes were observed under other conditions; the crystalline form of mesylate crystalline form I remained unchanged under all the above conditions. The results are summarized in Table 20.

**Table 20 Stability Test Results**

| Sample | Condition | Purity % | | | | Crystalline Form | | | Appearance | | | |
|---|---|---|---|---|---|---|---|---|---|---|---|---|
| | | day 0 | day 1 | day 3 | day 7 | day 1 | day 3 | day 7 | day 0 | day 1 | day 3 | day 7 |
| Citrate crystalline form I | 25°C/60%RH | 98.74 | - | 98.68 | 98.61 | - | Unchanged | Unchanged | Pale yellow solid | - | Unchanged | Unchanged |
| | 40°C/75%RH | | - | 98.66 | 98.59 | - | Unchanged | Unchanged | | - | Unchanged | Unchanged |
| | 25°C/95±5%RH | | - | 98.66 | 98.67 | - | Unchanged | Unchanged | | - | Unchanged | Unchanged |
| | 60 °C | | - | 98.68 | 98.66 | - | Unchanged | Unchanged | | - | Unchanged | Unchanged |
| | 80 °C | | 98.71 | 98.68 | - | Unchanged | Unchanged | - | | Unchanged | Unchanged | - |
| Maleate crystalline form II | 25°C/60%RH | 98.83 | - | 98.68 | 98.66 | - | Unchanged | Unchanged | Pale yellow solid | - | Unchanged | Unchanged |
| | 40°C/75%RH | | - | 98.67 | 98.64 | - | Unchanged | Unchanged | | - | Unchanged | Unchanged |
| | 25°C/95±5%RH | | - | 98.66 | 98.67 | - | Unchanged | Changed | | - | Unchanged | Unchanged |
| | 60 °C | | - | 98.66 | 98.68 | - | Unchanged | Unchanged | | - | Unchanged | Unchanged |
| | 80 °C | | 98.77 | 98.66 | - | Unchanged | Unchanged | - | | Unchanged | Unchanged | - |
| Mesylate crystalline form I | 25°C/60%RH | 98.50 | - | 98.19 | 98.24 | - | Unchanged | Unchanged | Pale yellow solid | - | Unchanged | Unchanged |
| | 40°C/75%RH | | - | 98.17 | 98.16 | - | Unchanged | Unchanged | | - | Unchanged | Unchanged |
| | 25°C/95±5%RH | | - | 98.17 | 98.20 | - | Unchanged | Unchanged | | - | Unchanged | Unchanged |
| | 60 °C | | - | 98.15 | 98.12 | - | Unchanged | Unchanged | | - | Unchanged | Unchanged |
| | 80 °C | | 98.38 | 98.24 | - | Unchanged | Unchanged | - | | Unchanged | Unchanged | - |

| | | | | | | | | | | | | |
|---|---|---|---|---|---|---|---|---|---|---|---|---|
| (a) Purity values above are mean of 3 parallel experiments. | | | | | | | | | | | | |

### 4.3 Pharmacokinetic Study

Pharmacokinetic studies in rats were performed for citrate crystalline form I, mesylate crystalline form I, and free base crystalline form I.

SD rats received a single oral gavage administration of citrate crystalline form I, mesylate crystalline form I, and free base crystalline form I, respectively. Blood samples were collected at various time points to determine drug concentrations in rat plasma post administration of tested substance and calculate PK-related parameters.

### 4.3.1 Preparation of test sample solutions

The preparation method was as follows: citrate crystalline form I, mesylate crystalline form I, and free base crystalline form I were accurately weighed, respectively, mixed with 5% DMSO, 10% solutol HS-15, and 85% saline by vortex, and set aside for later use.

### 4.3.2 Analysis of test sample solutions

The prepared test sample solutions were analyzed by the analytical department of the present test facility using LC-MS/MS.

### 4.3.3 Animal Reception and Acclimation

Animals were fasted (≥10 h) with free access to water from the day before dosing. On the test day, rats were weighed respectively, marked at the tail, and blank blood samples were collected before administration. Blood was collected via tail vein.

### 4.3.4 Sample Collection and Processing

Blood collection time points are:
Oral administration: 0.5, 1, 2, 4, 6, 8, 12, and 24 h post-dose.

0.1 mL of whole blood was collected into EDTA-Na2 anticoagulant tubes, inverted 3-4 times to mix well, centrifuged at 10,000 g, 4 °C for 5 min to separate plasma, and stored at - 80 °C until analysis. Blood was collected via tail vein.

Plasma samples were analyzed by the analytical department of the test facility using LC-MS/MS.

### 4.3.5 Pharmacokinetic Analysis

Based on the plasma concentration data of the drug, the pharmacokinetic parameters AUC₀₋ₗₐₛₜ, Cₘₐₓ, Tₘₐₓ, and T_{1/2} as well as their mean and standard deviation, were calculated using the non-compartmental model with WinNonlin pharmacokinetic calculation software.

### 4.3.6 Results

The main pharmacokinetic parameters in plasma after a single oral gavage of citrate crystalline form I, mesylate crystalline form I, and free base crystalline form I are shown in Table 21.

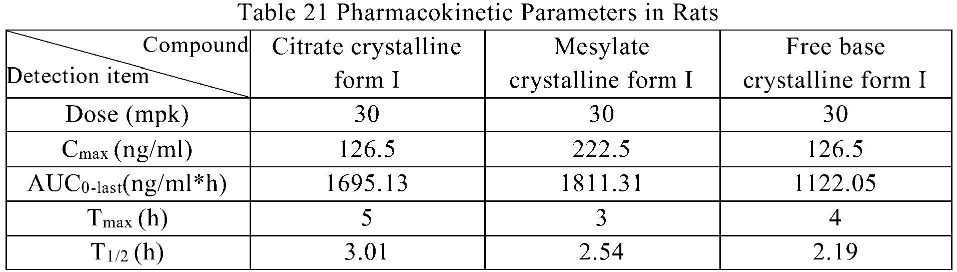

The PK results above demonstrate that *in vivo* exposure in rats of both citrate crystalline form I and mesylate crystalline form I was higher than that of free base crystalline form I.

In summary, citrate crystalline form I shows excellent solubility, high *in vivo* exposure, and excellent solid-state stability, making it suitable for further research and development.

All documents mentioned herein are incorporated by reference in this application as if each document were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. An acid salt crystalline form of a compound of Formula A, wherein the salt crystalline form is selected from the group consisting of: citrate crystalline form I, maleate crystalline form I, maleate crystalline form II, oxalate crystalline form I, oxalate crystalline form II, mesylate crystalline form I, and mesylate crystalline form II, 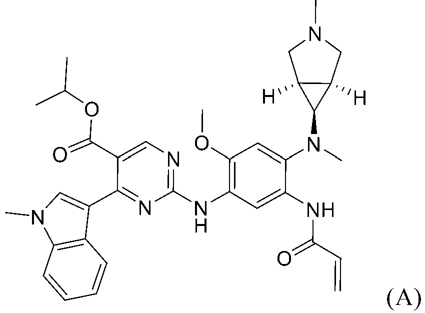 wherein,
the X-ray powder diffraction pattern of the citrate crystalline form I comprises characteristic peaks at 3 or more (e.g., 4, or 5) 20 values selected from the group consisting of: 6.9±0.2°, 7.9±0.2°, 13.3±0.2°, 16.0±0.2°, and 18.6±0.2°;
the X-ray powder diffraction pattern of the maleate crystalline form I comprises characteristic peaks at 3 or more (e.g., 4, or 5) 20 values selected from the group consisting of: 6.7±0.2°, 9.6±0.2°, 14.3±0.2°, 14.6±0.2°, and 23.5±0.2°;
the X-ray powder diffraction pattern of the maleate crystalline form II comprises characteristic peaks at 3 or more (e.g., 4, or 5) 20 values selected from the group consisting of: 6.9±0.2°, 10.6±0.2°, 14.5±0.2°, 16.5±0.2°, and 17.0±0.2°;
the X-ray powder diffraction pattern of the oxalate crystalline form I comprises characteristic peaks at 3 or more (e.g., 4, or 5) 20 values selected from the group consisting of: 9.4±0.2°, 12.0±0.2°, 12.7±0.2°, 13.7±0.2°, and 21.4±0.2°;
the X-ray powder diffraction pattern of the oxalate crystalline form II comprises characteristic peaks at 3 or more (e.g., 4, 5, 6, or 7) 20 values selected from the group consisting of: 9.5±0.2°, 12.2±0.2°, 12.7±0.2°, 13.7±0.2°, 14.5±0.2°, 19.9±0.2°, and 24.8±0.2°;
the X-ray powder diffraction pattern of the mesylate crystalline form I comprises characteristic peaks at 3 or more (e.g., 4, 5, or 6) 20 values selected from the group consisting of: 11.0±0.2°, 16.7±0.2°, 19.2±0.2°, 22.1±0.2°, 23.4±0.2°, and 25.0±0.2°; and
the X-ray powder diffraction pattern of the mesylate crystalline form II comprises characteristic peaks at 3 or more (e.g., 4, 5, or 6) 20 values selected from the group consisting of: 7.3±0.2°, 8.8±0.2°, 9.9±0.2°, 10.9±0.2°, 13.1±0.2°, and 14.2±0.2°.

2. The acid salt crystalline form according to claim 1, wherein
the X-ray powder diffraction pattern of the citrate crystalline form I comprises characteristic peaks at 5 or more (e.g., 6, 7, 8, or 9) 20 values selected from the group consisting of: 6.9±0.2°, 7.9±0.2°, 11.4±0.2°, 13.3±0.2°, 15.7±0.2°, 16.0±0.2°, 16.7±0.2°, 18.6±0.2°, 19.2±0.2°, and 20.8±0.2°;
the X-ray powder diffraction pattern of the maleate crystalline form I comprises characteristic peaks at 5 or more (e.g., 6, 7, 8, 9, or 10) 2θ values selected from the group consisting of: 6.7±0.2°, 8.1±0.2°, 9.6±0.2°, 14.3±0.2°, 14.6±0.2°, 15.8±0.2°, 16.4±0.2°, 20.2±0.2°, 22.4±0.2°, and 23.5±0.2°;
the X-ray powder diffraction pattern of the maleate crystalline form II comprises characteristic peaks at 5 or more (e.g., 6, 7, 8, 9 or 10) 2θ values selected from the group consisting of: 6.9±0.2°, 10.6±0.2°, 14.5±0.2°, 15.5±0.2°, 16.1±0.2°, 16.5±0.2°, 17.0±0.2°, 19.4±0.2°, 21.5±0.2°, and 22.0±0.2°;
the X-ray powder diffraction pattern of the oxalate crystalline form I comprises characteristic peaks at 5 or more (e.g., 6, 7, 8, 9, 10 or 11) 2θ values selected from the group consisting of: 6.8±0.2°, 9.4±0.2°, 12.0±0.2°, 12.7±0.2°, 13.7±0.2°, 14.0±0.2°, 19.9±0.2°, 20.6±0.2°, 21.4±0.2°, 22.5±0.2°, and 24.9±0.2°;
the X-ray powder diffraction pattern of the oxalate crystalline form II comprises characteristic peaks at 5 or more (e.g., 6, 7, 8, 9, 10 or 11) 2θ values selected from the group consisting of: 9.1±0.2°, 9.5±0.2°, 10.6±0.2°, 12.2±0.2°, 12.7±0.2°, 13.7±0.2°, 14.3±0.2°, 14.5±0.2°, 19.9±0.2°, 21.9±0.2°, 23.1±0.2°, and 24.8±0.2°;
the X-ray powder diffraction pattern of the mesylate crystalline form I comprises characteristic peaks at 5 or more (e.g., 6, 7, 8, 9, 10 or 11) 20 values selected from the group consisting of: 8.8±0.2°, 10.2±0.2°, 11.0±0.2°, 15.6±0.2°, 16.7±0.2°, 17.0±0.2°, 18.5±0.2°, 19.2±0.2°, 22.1±0.2°, 23.4±0.2°, and 25.0±0.2°; and
the X-ray powder diffraction pattern of the mesylate crystalline form II comprises characteristic peaks at 5 or more (e.g., 6, 7, 8, 9, or 10) 2θ values selected from the group consisting of: 7.2±0.2°, 7.3±0.2°, 8.8±0.2°, 9.9±0.2°, 10.9±0.2°, 13.1±0.2°, 14.2±0.2°, 16.6±0.2°, 16.9±0.2°, and 22.1±0.2°.

3. The acid salt crystalline form according to claim 1, wherein
the X-ray powder diffraction pattern of the citrate crystalline form I comprises characteristic peaks at 6 or more (e.g., 7, 8, 9, or 10) 20 values selected from the group consisting of: 6.9±0.2°, 7.9±0.2°, 11.4±0.2°, 13.3±0.2°, 14.9±0.2°, 15.7±0.2°, 16.0±0.2°, 16.7±0.2°, 17.2±0.2°, 18.6±0.2°, 19.2±0.2°, 20.8±0.2°, 21.0±0.2°, 22.9±0.2°, 23.8±0.2°, and 27.1±0.2°;
the X-ray powder diffraction pattern of the maleate crystalline form I comprises characteristic peaks at 6 or more (e.g., 7, 8, 9, 10, 11 or 12) 2θ values selected from the group consisting of: 6.7±0.2°, 8.1±0.2°, 9.6±0.2°, 14.3±0.2°, 14.6±0.2°, 15.8±0.2°, 16.4±0.2°, 19.4±0.2°, 19.9±0.2°, 20.2±0.2°, 21.4±0.2°, 22.4±0.2°, 23.5±0.2°, and 24.6±0.2°;
the X-ray powder diffraction pattern of the maleate crystalline form II comprises characteristic peaks at 6 or more (e.g., 7, 8, 9, 10, 11 or 12) 2θ values selected from the group consisting of: 6.9±0.2°, 9.8±0.2°, 10.6±0.2°, 14.5±0.2°, 15.5±0.2°, 16.1±0.2°, 16.5±0.2°, 17.0±0.2°, 19.4±0.2°, 21.5±0.2°, 22.0±0.2°, 23.1±0.2°, 25.2±0.2°, 25.8±0.2°, and 27.3±0.2°;
the X-ray powder diffraction pattern of the oxalate crystalline form I comprises characteristic peaks at 6 or more (e.g., 7, 8, 9, or 10) 20 values selected from the group consisting of: 6.8±0.2°, 9.0±0.2°, 9.4±0.2°, 10.6±0.2°, 12.0±0.2°, 12.7±0.2°, 13.7±0.2°, 14.0±0.2°, 14.2±0.2°, 15.3±0.2°, 19.1±0.2°, 19.9±0.2°, 20.6±0.2°, 21.4±0.2°, 22.5±0.2°, 23.6±0.2°, and 24.9±0.2°;
the X-ray powder diffraction pattern of the oxalate crystalline form II comprises characteristic peaks at 6 or more (e.g., 7, 8, 9, 10, 11 or 12) 20 values selected from the group consisting of: 6.8±0.2°, 9.1±0.2°, 9.5±0.2°, 10.6±0.2°, 12.2±0.2°, 12.7±0.2°, 13.7±0.2°, 13.8±0.2°, 14.3±0.2°, 14.5±0.2°, 19.9±0.2°, 21.1±0.2°, 21.9±0.2°, 23.1±0.2°, 23.8±0.2°, and 24.8±0.2°;
the X-ray powder diffraction pattern of the mesylate crystalline form I comprises characteristic peaks at 6 or more (e.g., 7, 8, 9, 10, 11 or 12) 20 values selected from the group consisting of: 8.8±0.2°, 10.2±0.2°, 11.0±0.2°, 15.6±0.2°, 16.7±0.2°, 17.0±0.2°, 17.6±0.2°, 18.5±0.2°, 19.2±0.2°, 19.6±0.2°, 20.3±0.2°, 21.5±0.2°, 22.1±0.2°, 22.7±0.2°, 23.4±0.2°, and 25.0±0.2°; and
the X-ray powder diffraction pattern of the mesylate crystalline form II comprises characteristic peaks at 6 or more (e.g., 7, 8, 9, 10, 11 or 12) 2θ values selected from the group consisting of: 7.2±0.2°, 7.3±0.2°, 8.8±0.2°, 9.9±0.2°, 10.9±0.2°, 12.5±0.2°, 13.1±0.2°, 14.2±0.2°, 14.9±0.2°, 16.6±0.2°, 16.9±0.2°, 22.1±0.2°, 22.6±0.2°, and 23.4±0.2°.

4. The acid salt crystalline form according to claim 1, wherein
the X-ray powder diffraction pattern of the citrate crystalline form I is substantially as shown in Figure 1;
the X-ray powder diffraction pattern of the maleate crystalline form I is substantially as shown in Figure 6;
the X-ray powder diffraction pattern of the maleate crystalline form II is substantially as shown in Figure 8;
the X-ray powder diffraction pattern of the oxalate crystalline form I is substantially as shown in Figure 10;
the X-ray powder diffraction pattern of the oxalate crystalline form II is substantially as shown in Figure 12;
the X-ray powder diffraction pattern of the mesylate crystalline form I is substantially as shown in Figure 14; and
the X-ray powder diffraction pattern of the mesylate crystalline form II is substantially as shown in Figure 16.

5. The acid salt crystalline form according to claim 1, wherein the thermogravimetric analysis pattern and the differential scanning calorimetry pattern of the citrate crystalline form I are substantially as shown in Figure 2;
the thermogravimetric analysis pattern and the differential scanning calorimetry pattern of the maleate crystalline form I are substantially as shown in Figure 7;
the thermogravimetric analysis pattern and the differential scanning calorimetry pattern of the maleate crystalline form II are substantially as shown in Figure 9;
the thermogravimetric analysis pattern and the differential scanning calorimetry pattern of the oxalate crystalline form I are substantially as shown in Figure 11;
the thermogravimetric analysis pattern and the differential scanning calorimetry pattern of the oxalate crystalline form II are substantially as shown in Figure 13;
the thermogravimetric analysis pattern and the differential scanning calorimetry pattern of mesylate crystalline form I are substantially as shown in Figure 15; and
the thermogravimetric analysis pattern and the differential scanning calorimetry pattern of mesylate crystalline form II are substantially as shown in Figure 17;
more preferably, the citrate crystalline form I is triclinic system, space group P-1, the unit cell parameters are a = 11.9331(2) Å, b = 13.4684(2) Å, c = 13.6474(2) Å, α = 69.2800(10)°, β = 82.1490(10)°, γ = 85.5730(10)°, V = 2031.32(6) Å³, Z = 2.

6. The acid salt crystalline form according to claim 1, wherein in the citrate crystalline form I, the molar ratio of the compound of Formula A to citric acid is (0.8-1.2):(0.8-1.2), preferably (0.9-1.1):(0.9-1.1);
in the maleate crystalline form I, the molar ratio of the compound of Formula A to maleic acid is (0.8-1.2):(0.8-1.2), preferably (0.9-1.1):(0.9-1.1);
in the maleate crystalline form II, the molar ratio of the compound of Formula A to maleic acid is (0.8-1.2):(0.8-1.2), preferably (0.9-1.1):(0.9-1.1);
in the oxalate crystalline form I, the molar ratio of the compound of Formula A to oxalic acid is (0.8-1.2):(0.8-1.2), preferably (0.9-1.1):(0.9-1.1);
in the oxalate crystalline form I, the molar ratio of the compound of Formula A to oxalic acid is (0.8-1.2):(0.8-1.2), preferably (0.9-1.1):(0.9-1.1);
in the mesylate crystalline form I, the molar ratio of the compound of Formula A to methanesulfonic acid is (0.9-1.1):(0.9-1.1); and
in the mesylate crystalline form II, the molar ratio of the compound of Formula A to methanesulfonic acid is (0.9-1.1):(0.9-1.1).

7. A method for preparing the acid salt crystalline form of the compound of Formula A according to claim 1, wherein the method is selected from the group consisting of:
method 1:
mixing the compound of Formula A with a first solvent, adding a first acid reagent, and crystallizing out; the first solvent is one selected from the group consisting of methanol, acetone, and acetonitrile;
method 2:
(a) mixing the compound of Formula A with a second solvent, then mixing with a second acid reagent to obtain an acid salt of the compound of Formula A; the second solvent is one selected from the group consisting of 1,2-dichloroethane, methanol, acetone, and acetonitrile;
(b) mixing the acid salt of the compound of Formula A with a third solvent, subjecting to polymorphic transition in suspension, and crystallizing out; the third solvent is one or more selected from the group consisting of dichloromethane, acetone, tetrahydrofuran, acetonitrile, and methyl *tert-butyl* ether;
and method 3:
(c) heating an acid salt crystalline form of the compound of Formula A to a certain temperature, performing polymorphic transition to obtain another crystalline form.

8. A pharmaceutical composition, wherein the pharmaceutical composition comprises an acid salt crystalline form of the compound of Formula A according to claim 1, and pharmaceutically acceptable carriers.

9. Use of the acid salt crystalline form of the compound of Formula A according to any one of claims 1-6 for the manufacture of a medicament, wherein the medicament is used for the uses selected from the group consisting of:
1) preventing and/or treating diseases or disorders associated with modulation of EGFR and/or HER2;
2) preventing and/or treating tumors; and
3) inhibiting cell proliferation.

10. The use according to claim 9, wherein the tumor is one or more of breast cancer, cervical cancer, colon cancer, lung cancer, gastric cancer, rectal cancer, pancreatic cancer, brain cancer, skin cancer, oral cancer, prostate cancer, bone cancer, renal cancer, ovarian cancer, bladder cancer, liver cancer, fallopian tube tumor, peritoneal tumor, melanoma, glioma, neuroglioblastoma, head and neck cancer, papillary renal tumor, leukemia, lymphoma, myeloma, and thyroid tumor.
